# EUROPEAN PATENT APPLICATION

(11) **EP 4 663 757 A1**
(43) Date of publication of application: **17.12.2025**
(21) Application number: 24753443.1
(22) Date of filing: 09.02.2024
(51) Int. Cl.: C12N 15/113, A61K 31/7088, A61K 48/00, A61P 25/00, A61P 25/08, A61P 25/16, A61P 25/28, A61P 27/02, A61P 27/06

(54) **TAU SPLICE-SWITCHING OLIGONUCLEOTIDE, AND PHARMACEUTICAL COMPOSITION FOR TREATING OR PREVENTING TAU-RELATED DISEASE**

(30) Priority: 10.02.2023 JP 2023019305
(71) Applicant: Otsuka Pharmaceutical Co., Ltd., Tokyo 101-8535 (JP)
(72) Inventor: SUZUKI, Shunya, Osaka-shi, Osaka 540-0021 (JP); NUKAGA, Yohei, Osaka-shi, Osaka 540-0021 (JP); KUNIYOSHI, Yuki, Osaka-shi, Osaka 540-0021 (JP); FUTAMURA, Takashi, Osaka-shi, Osaka 540-0021 (JP)
(74) Representative: Hoffmann Eitle
(86) International application number: PCT/JP2024/004556
(87) International publication number: WO 2024/167000

(57) **Abstract**

The present disclosure includes a splice-switching oligonucleotide that binds to intron 1B of TAU pre-mRNA, and a pharmaceutical composition comprising the splice-switching oligonucleotide for treating or preventing a TAU-related disease.

## Description

### TECHNICAL FIELD

This application claims benefit of priority to Japanese Patent Application 2023-019305, the entire content of which is incorporated herein by reference.

The present disclosure includes a splice-switching oligonucleotide directed to TAU pre-mRNA, and a pharmaceutical composition comprising the splice-switching oligonucleotide for treating or preventing a TAU-related disease.

### BACKGROUND

TAU, a microtubule-binding protein, is abundantly expressed in neurons of the central nervous system and it is involved in microtubule stabilization of axons. Abnormalities in TAU lead to axonal degeneration, neurofibrillary tangle, and abnormal localization of TAU, resulting in diseases such as tauopathy. Many antisense oligonucleotides called gapmers have been developed to reduce TAU expression. A gapmer regulates protein expression of the target by inducing cleavage of RNA of the target by ribonuclease H (RNase H) relying on DNA/RNA double-strand.

Splicing is a process removing introns from mRNA precursor (pre-mRNA) transcribed from a gene and joining exons together to provide mature mRNA encoding a protein from the pre-mRNA. Splicing modulators, which regulate the splicing and restore functional protein, have been developed and commercialized for the treatment of spinal muscular atrophy. To date, however, there is no marketed agent that reduces TAU expression by regulating splicing.

### SUMMARY OF THE INVENTION

### PROBLEMS TO BE SOLVED BY THE INVENTION

An object of the present disclosure is to provide a splice-switching oligonucleotide directed to TAU pre-mRNA and a pharmaceutical composition comprising the splice-switching oligonucleotide for treating or preventing a TAU-related disease.

### SOLUTIONS TO THE PROBLEMS

In one aspect, the present disclosure provides a splice-switching oligonucleotide that binds to intron 1B of TAU pre-mRNA.

In one aspect, the present disclosure provides a pharmaceutical composition comprising the splice-switching oligonucleotide for treating or preventing a TAU-related disease.

### EFFECTS OF THE INVENTION

The present disclosure enables the treatment or prevention of a TAU-related disease by modulating splicing of TAU pre-mRNA and reducing TAU expression.

### BRIEF DESCRIPTION OF THE DRAWINGS

[Fig. 1A] Identification of sense sequence which is used to promote inclusion of exon 1B (1). The sense strand sequence of TAU pre-mRNA to which oligonucleotides hybridize (SEQ ID NO: 38), positions of primers (118, 120) used for RT-PCR, and representative TAU transcripts are shown.
[Fig. 1B] Identification of sense sequence which is used to promote inclusion of exon 1B (2). iPS neurons were treated with 1 µM of an oligonucleotide (comparative example compound 1, Scramble, compounds 2 to 5) for 15 days. Then, RT-PCR was performed for TAU (top) and ACTB as a control (bottom). The positive control for PCR was a synthetic DNA template (right end). The expected transcripts from the molecular weights are shown on the right.
[Fig. 2A] Confirmation of exon structure by Sanger sequencing (1). The three amplicons, which were derived from the compound 4-treated sample, at around 335bp, 235bp, and 200bp in Fig. 1B were extracted from the gel and cloned into plasmids, respectively. Each plasmid was checked by using PCR and electrophoresis with respect to whether an insert existed in the plasmid.
[Fig. 2B] Confirmation of exon structure by Sanger sequencing (2). The base sequence of clone No. 2 in Fig. 2A (top) was evaluated by Sanger sequencing. The result of a single end analysis with a forward primer is shown. The termination codon (TGA) included in exon 1B is highlighted with an underline and "STOP".
[Fig. 2C] Confirmation of exon structure by Sanger sequencing (3). The base sequence of clone No. 1 0 in Fig. 2A (middle) was evaluated by Sanger sequencing. The result of a single end analysis with a forward primer is shown. The termination codon (TGA) included in exon 1B is highlighted with an underline and "STOP".
[Fig. 2D] Confirmation of exon structure by Sanger sequencing (4). The base sequence of clone No. 24 in Fig. 2A (bottom) was evaluated by Sanger sequencing. The result of a single end analysis with a forward primer is shown.
[Fig. 3A] Establishment of qPCR selectively quantifying TAU productive mRNA or TAU nonproductive mRNA (1). (Top) The positions of primers (arrows) and a probe (line) designed to selectively detect TAU nonproductive mRNA are shown. (Bottom) The graph shows Ct values and copy numbers obtained from serially-diluted synthetic DNA templates by performing qPCR with the reagent set for TAU nonproductive mRNA detection. When the Ct value was greater than 40 in 10⁶ copies, it was noted as ND.
[Fig. 3B] Establishment of qPCR selectively quantifying TAU productive mRNA or TAU nonproductive mRNA (2). (Top) The positions of primers (arrows) and a probe (line) designed to selectively detect TAU productive mRNA are shown. (Bottom) The graph shows Ct values and copy numbers obtained from serially-diluted synthetic DNA templates by performing qPCR with the reagent set for TAU productive mRNA detection.
[Fig. 3C] Establishment of qPCR selectively quantifyingTAU productive mRNA or TAU nonproductive mRNA (3). (Top) The positions of primers (arrows) and a probe (line) designed to selectively detect TAU pre-mRNA are shown. (Bottom) The graph shows Ct values and copy numbers obtained from serially-diluted synthetic DNA templates by performing qPCR with the reagent set for TAU pre-mRNA detection.
[Fig. 4A] Examination of splice-switching activity of oligonucleotides (1). The sense strand sequence of TAU pre-mRNA to which compounds 1 to 6 hybridize (SEQ ID NO: 38) and TAU transcripts (productive and nonproductive mRNAs including exon 4) are shown in schematic representation.
[Fig. 4B] Examination of splice-switching activity of oligonucleotides (2). iPS neurons were treated with 1 µM of an oligonucleotide (Scramble, comparative example compound 1, compounds 1 to 6) for 15 days, and the copy numbers of nonproductive mRNA, productive mRNA and ACTB were quantified. After being normalized by the value of ACTB, the results were plotted on a graph by adjusting the result of the non-treated group to 100%.

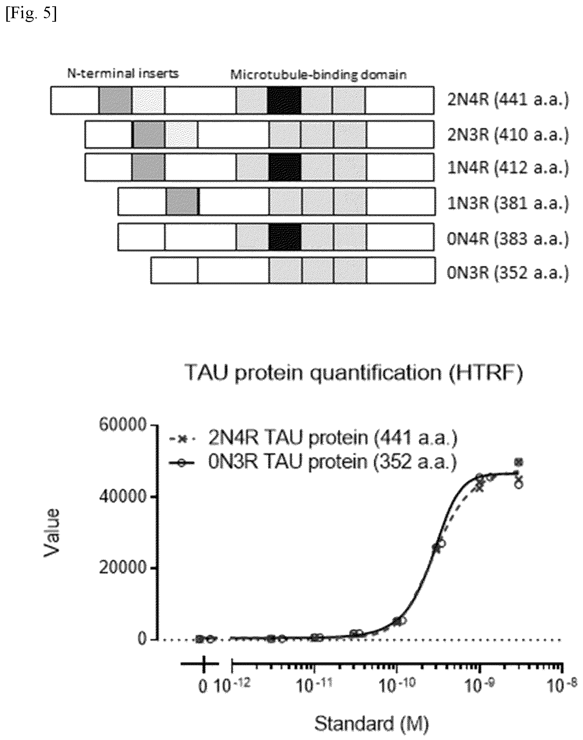

[Fig. 5] Quantification of TAU protein. (Top) The variants of TAU protein expressed in the central nervous system are shown. There are six splicing variants, i.e., combinations of 2, 1, or 0 N-terminal insert(s) and 4 or 3 C-terminal microtubule-binding domain inserts. Common abbreviations and the number of amino acids are shown. (Bottom) The longest 2N4R TAU recombinant protein (cross, dotted line) and the shortest 0N3R TAU recombinant protein (circle, solid line) were quantified by using a commercially available HTRF^{®} kit, and the concentration of the recombinant protein and the signals of fluorescence obtained were plotted on the graph.
[Fig. 6A] Study of concentration dependency (I) (1). The iPS neurons were treated with compound 7 at a given concentration for 15 days, and expression level of each target was quantified.
[Fig. 6B] Study of concentration dependency (I) (2). The iPS neurons were treated with compound 8 at a given concentration for 15 days, and expression level of each target was quantified.
[Fig. 6C] Study of concentration dependency (I) (3). The iPS neurons were treated with a gapmer (comparative example compound 1) at a given concentration for 15 days, and expression level of each target was quantified.
[Fig. 7A] Identification of neuronal cells with increased TAU nonproductive mRNA (1). TAU nonproductive mRNA was visualized after iPS neurons were treated with compound 7 or 8 at 3 µM for 15 days. The scale bar indicates 10 µm.
[Fig. 7B] Identification of neuronal cells with increased TAU nonproductive mRNA (2). After the procedures of Fig. 7A, the cells were immunostained with antibodies against NeuN, a marker of mature neurons, and GABA, a marker of inhibitory neurons. The scale bar indicates 10 µm.
[Fig. 8A] scRNA-seq (1). The schedule of the treatment of iPS neurons with an oligonucleotide is shown.
[Fig. 8B] scRNA-seq (2). The result of clustering into two dimensions by compressing dimensions in the gene expression profiles of each cell is shown.
[Fig. 8C] scRNA-seq (3). The heat map of marker gene expression in each cluster is shown.
[Fig. 8D] scRNA-seq (4). The violin plots of TAU (MAPT) expression in the population of excitatory or inhibitory neurons treated with a gapmer (comparative example compound 1), compound 7, or compound 8 are shown.
[Fig. 8E] scRNA-seq (5). The mean value and 95% confidence interval for the delta in the percentage of reduced TAU (MAPT) expression between the compound-treated groups of excitatory and inhibitory neurons were calculated with the bootstrap method and are shown.
[Fig. 9A] Attenuation of hyper synchronization of neural activity by reduction of endogenous TAU protein (1). The schedule of the oligonucleotide treatment on the culture of iPS neurons and iPS astrocytes is shown.
[Fig. 9B] Attenuation of hyper synchronization of neural activity by reduction of endogenous TAU protein (2). Raster plots of pre (left) and post (right) KCl treatments are shown. Individual thin lines: spikes, individual bold lines: bursts (= resulting from prolonged spikes for several seconds), squares: network bursts, spines: spike histograms.
[Fig. 9C] Attenuation of hyper synchronization of neural activity by reduction of endogenous TAU protein (3). The number of network bursts in pretreatment was normalized to 100%, and the number of network bursts observed in posttreatment was shown in percentage against it. N = 6 wells. Paired T-tests: *<0.05, **<0.01.
[Fig. 10A] Study of concentration dependency (II) (1). The iPS neurons were treated with compound 7 at a given concentration for 15 days, and expression level of each target was quantified.
[Fig. 10B] Study of concentration dependency (II) (2). The iPS neurons were treated with compound 8 at a given concentration for 15 days, and expression level of each target was quantified.
[Fig. 10C] Study of concentration dependency (II) (3). The iPS neurons were treated with a gapmer (comparative example compound 2) at a given concentration for 15 days, and expression level of each target was quantified.
[Fig. 10D] Study of concentration dependency (II) (4). The iPS neurons were treated with a gapmer (comparative example compound 3) at a given concentration for 15 days, and expression level of each target was quantified.

### DETAILED DESCRIPTION

Unless otherwise defined, the terms used herein are read as generally understood by those skilled in the technical fields such as organic chemistry, medical sciences, pharmaceutical sciences, molecular biology, and microbiology. Several terms used herein are defined as below. The definitions herein take precedence over the general understandings.

The present disclosure relates to an oligonucleotides capable of reducing an amount of TAU protein by modulating splicing of TAU pre-mRNA.

TAU is a microtubule-binding protein abundantly expressed in neurons of the central nervous system and it is involved in microtubule stabilization of axons. TAU has four regions from the N-terminus: N-terminal region, proline-rich region, microtubule-binding region, and C-terminal region. The human TAU gene is located on chromosome 17 and is also called microtubule-associated protein tau (MAPT) gene. TAU pre-mRNA refers to mRNA that has been transcribed from the TAU gene and has not undergone splicing. The human TAU gene is reported to have at least 16 exons, and alternative splicing of TAU pre-mRNA results in six different TAU protein variants ranging from 352 to 441 amino acids in length in humans. The N-terminal region has two inserts, N1 and N2, encoded by exons 2 and 3, respectively. The microtubule-binding region is divided into four domains, R1 to R4, and R2 is encoded by exon 10. There are three types of N-terminal region: type 0N, which lacks both exons 2 and 3; type 1N, which includes only exon 2; and type 2N, which includes both exons 2 and 3. There are two types of microtubule-binding region: type 3R, which lacks exon 10; and type 4R, which includes exon 10. These types in combination provides six variants.

Splicing is a process that removes introns from pre-mRNA transcribed from a gene and joins exons. For splicing, it has been known that an exonic splicing enhancer (ESE) sequence, exonic splicing silencer (ESS) sequence, intronic splicing enhancer (ISE) sequence, or intronic splicing silencer (ISS) sequence in an exon or intron is recognized by an RNA-binding protein (RBP) and regulates skipping or inclusion (insertion) of an exon or intron.

The inventors focused on a transcript registered in the database as ENST00000571311.5, in which 135 nucleotides (nt) that should have been recognized as a part of introns (SEQ ID NO: 1) were recognized as an exon and included between exons 1 and 2. A region corresponding to this included sequence on DNA or RNA is herein referred to as exon 1B. Since a premature termination codon appears due to the inclusion of exon 1B, no TAU protein is produced from mRNA including exon 1B. This exon 1B-including mRNA is assumed to be degraded via NMD (nonsense-mediated mRNA decay). In the present disclosure, mRNA that produces the protein encoded by the gene is also referred to as productive mRNA, and mRNA that does not produce the protein encoded by the gene is also referred to as nonproductive mRNA.

As used herein, exon 1B of TAU pre-mRNA (also referred to as TAU exon 1B) refers to a region of TAU pre-mRNA that corresponds to the sequence of SEQ ID NO: 1 when the TAU pre-mRNA and the sequence of SEQ ID NO: 1 are optimally aligned (aligned to achieve maximum matches), and it is not limited to a region consisting of the sequence of SEQ ID NO: 1. As used herein, when a base sequence represents an RNA sequence, the letter T in the base sequence is understood to mean the letter U. When an RNA sequence is aligned with a base sequence described herein, the letter T in the base sequence is considered as the letter U.

### TAU exon 1B

AGGGGAGTGAATTCACTGAGCTGAGAGCTGAGGAACCATTGATCTGATGGCT
GAGACACCACTGGGAAGACTGGAGAGGCTTTTCTGGGCATGCAGTGCCAGG
CACAGGAGGAGCTGAGGGAAGATGACTAAGAG (SEQ ID NO: 1)

The oligonucleotide of the present disclosure can bind to TAU pre-mRNA and modulate its splicing, and as a result, increase the amount of TAU mRNA including exon 1B and reduce the amount of TAU protein. An oligonucleotide binds to pre-mRNA through hydrogen bonds between the bases of its nucleosides and those of the nucleosides of pre-mRNA complementary thereto. An oligonucleotide that can bind to pre-mRNA refers to an oligonucleotide that is complementary enough to form a duplex with the pre-mRNA, and the oligonucleotide need not have a sequence that is completely complementary to the pre-mRNA. Complementary base pairs include adenine (A) and thymine (T), adenine (A) and uracil (U), and cytosine (C) and guanine (G).

In the present disclosure, an oligonucleotide that can modulate splicing is referred to as a splice-switching oligonucleotide (SSO). That is, the term "oligonucleotide" herein encompasses an SSO, and descriptions to an "oligonucleotide" herein are also applied to an SSO. An oligonucleotide that functions as an SSO does not induce degradation of TAU pre-mRNA by RNase H. RNase H is an enzyme that specifically cleaves RNA strand of DNA/RNA double-strand. Different from an SSO, an antisense oligonucleotide called gapmer induces degradation of target mRNA by RNase H. When nonproductive mRNA increases while productive mRNA decreases in oligonucleotide-treated cells, as described in Experiment 5 of the examples, it is understood that the oligonucleotide functions as an SSO and does not induce TAU pre-m RNA degradation by RNase H.

As used herein, the intron between exon 1B and exon 2 is referred to as intron 1B. The oligonucleotide of the present disclosure binds to intron 1B of TAU pre-mRNA. The oligonucleotide may bind to any region of intron 1B as long as it can modulate splicing to increase the amount of TAU mRNA including exon 1B. A representative sequence of the sense strand of intron 1B of TAU pre-mRNA is shown in SEQ ID NO: 2. Intron 1B of TAU pre-mRNA refers to a region of TAU pre-mRNA that corresponds to the sequence of SEQ ID NO: 2 when the TAU pre-mRNA and the sequence of SEQ ID NO: 2 are optimally aligned (aligned to achieve maximum matches), and it is not limited to a region consisting of the sequence of SEQ ID NO: 2. In some embodiments, the oligonucleotide comprises or consists of a sequence complementary to a part of intron 1B of TAU pre-mRNA.

### TAU intron 1B

GTACTGGCAAAGAATTCAGAAATTCTGATGGAAGCTTTACATGTTACCATCAC
ATCCATCCATCTATCCACCCATCCATCCACCCATATCTTCCTCCCTCCACCCAA
TCATGCATACATCCAGTCATCTATACACCACCCACCCACCCATCCATCCATCCA
TCCATCCCTTCATCCATCCCATCATCCATCCAATTATACATACATCCAATCATAT
ATCTGTACATAATCCATTCTTCCCTCGGTTCATCCATCCATCCATTCATCCATCC
ATCCACCCATCCCTTCCTTCATCCTTCCTATCATCCATCCAATCATATATCTGTA
CATAATCCATTCTTCCCTCGGTTCATCCATCCATCCATTCATCCATCCATCCACC
CATCCCTTCCTTCATCCTTCCTATCATCCATCCAATCATACATATATCCAATCAT
ACATCTGCACATCACCAGCTCATCCATCTATCCATTTATCCATCCATCCTTCCTT
CCATCCATCATTCATCCATCATACATACATCTAACCATACATCTCTACATCATTC
ATTCTTCCATCGATTCATCCAATTATCCATCATTCCTTCCTCCATCCATCCCATT
ATCCATTTGATCATACATATATCATCTATACATCATCCATTCATCCATCCATCCAT
CCATCCACCCATATCTTCATCCAATCAATCATACATACATCGAATCATCTACAC
ATCACCCATCCATCCATCCATCCATTCATCTATCCACCCATCCATCCATCCATCC
ATCCATTCATCTATCCACCCATCCATCCATCCATCCATCCATCCATCCATGTAAC
CATCCAGTCATATATCCAATTACACATCCATCCAGTTATACATTCATACATGCAT
CTAATCATTCAATTATACATACACACATCCATATAATTCTACATCCAATTATACC
TCCATCCAATTACACATTCATACACCCACCTAATAAATTATTAATTCATATATCC
ATCCATATAATTATACATCAATTATACATCCATCTAATCATTCAGTAATTCACCC
ACCATCCAGTCATCTATCCAATAATACATTCATCCAATCATCCATCCATCCATCC
ACCCATTCATCCATCCATCCGTCCGTCCACCCATCATGGTATGAGCCATGATTT
ACCACGATGGTCCCCTGTGGACAGCCCAGGTGGGGCAGAACTGAAGGGAAG
CCCAGGGCTGCCCCCATAAACATTTGCCTCCTTTACATGGATGAGAACTAGAT

CCACATGTATAAATCCTCATGATTTGAAGGTGCTTTTACCAACATTCACTCATG
GGATTCTCCCAGGAGCTCTAGGAGGAGGCAGGTAGAGTTGAGGTCATCTCAC
GCATTTTACAGATGAGGAAACGGAGGCCCTGAGAGGCAGGTCCAAGGCCAC
CTGACCAGAAAGAAGTGGAACTGGGACTTGAACCCAGCCATCTTGCCCCTT
GGTCCCATGCTCTCTAGCCTGTAACTCCTGCTTCCTGGTGGGGCATCTCCAGG
AGGACCCTATCGGCTGGCCATGGGCCTGCCCTGGAGTCTTTTGCTCTGTGTG
GCCATCCTTCCTCCCTCAGGAGAGTGTGTGCTCCCAGAGCACAGGCTGTATC
TTCTGAGCATTTTGTCCCTTCCCAGTACCTAGCACTCAGCTCTGTATACATTGG
GCTCTCAAGAATTCTCAACCTTCCAGAGTGTAAGGCCTTGACCTGCTCAGCC
CTGGATACTGCATGATGCATTGATAAGCCCATAAAATAACCAGGGCAGATTGA
CTCCCAGTGGCCAAAGTGCCACAGGGAAGGGACAATTCAGCCCTTCTAGGA
GGAGGAGGAGGTAGTTTTCTCATTTCTATTAAGGCAACAAAAGCTGCCTTAC
TAAGGACATTCTTGGTGGAGGGCGTGACTGTCAACCACTGTGATCATTTGGG
CCTCTCTTGCCCAGGCTTCCCATTCTGAAAGGACAGTTTTATTGTAGGTACAC
ATGGCTGCCATTTCAAATGTAACTCACAGCTTGTCCATCAGTCCTTGGAGGTC
TTTCTATGAAAGGAGCTTGGTGGCGTCCAAACACCACCCAATGTCCACTTAG
AAGTAAGCACCGTGTCTGCCCTGAGCTGACTCCTTTTCCAAGGAAGGGGTTG
GATCGCTGAGTGTTTTTCCAGGTGTCTACTTGTTGTTAATTAATAGCAATGAC
AAAGCAGAAGGTTCATGCGTAGCTCGGCTTTCTGGTATTTGCTGCCCGTTGA
CCAATGGAAGATAAACCTTTGCCTCAGGTGGCACCACTAGCTGGTTAAGAGG
CACTTTGTCCTTTCACCCAGGAGCAAACGCACATCACCTGTGTCCTCATCTG
ATGGCCCTGGTGTGGGGCACAGTCGTGTTGGCAGGGAGGGAGGTGGGGTTG
GTCCCCTTTGTGGGTTTGTTGCGAGGCCGTGTTCCAGCTGTTTCCACAGGGA
GCGATTTTCAGCTCCACAGGACACTGCTCCCCAGTTCCTCCTGAGAACAAAA
GGGGGCGCTGGGGAGAGGCCACCGTTCTGAGGGCTCACTGTATGTGTTCCA
G (SEQ ID NO: 2)

In some embodiments, the oligonucleotide comprises or consists of:
a sequence complementary to a sequence having 70%, 75%, 80%, 85%, 90%, or 95% or more sequence identity to the sequence of 5'-GGCAAAGAATTCAGAAATT-3' (SEQ ID NO: 3); or
a sequence complementary to the sequence of 5'-GGCAAAGAATTCAGAAATT-3' (SEQ ID NO: 3), or a sequence complementary to a sequence that differs from the sequence of SEQ ID NO: 3 in that 1 to 6, 1 to 5, 1 to 4, 1 to 3, 1 or 2 bases are modified in the sequence of SEQ ID NO: 3.

The sequence of SEQ ID NO: 3 corresponds to the sequence at positions 6 to 24 of the sequence of the sense strand of intron 1B of TAU pre-mRNA represented by SEQ ID NO: 2.

In some embodiments, the oligonucleotide comprises or consists of a sequence complementary to a sequence consisting of at least 13 consecutive bases (i.e., 13, 14, 15, 16, 17, 18, or 19 bases) in the sequence of 5'-GGCAAAGAATTCAGAAATT-3' (SEQ ID NO: 3).

In some embodiments, the oligonucleotide comprises or consists of:
a sequence complementary to a sequence having 70%, 75%, 80%, 85%, 90%, or 95% or more sequence identity to the sequence of 5'-GGCAAAGAATTCAGAAAT-3' (SEQ ID NO: 4) or 5'-GCAAAGAATTCAGAAATT-3' (SEQ ID NO: 5); or
a sequence complementary to the sequence of 5'-GGCAAAGAATTCAGAAAT-3' (SEQ ID NO: 4) or 5'-GCAAAGAATTCAGAAATT-3' (SEQ ID NO: 5), or a sequence complementary to a sequence that differs from the sequence of SEQ ID NO: 4 or SEQ ID NO: 5 in that 1 to 6, 1 to 5, 1 to 4, 1 to 3, 1 or 2 base(s) are modified in the sequence of SEQ ID NO: 4 or SEQ ID NO: 5.

In some embodiments, the oligonucleotide comprises or consists of a sequence complementary to the sequence of 5'-GGCAAAGAATTCAGAAAT-3' (SEQ ID NO: 4) or 5'-GCAAAGAATTCAGAAATT-3' (SEQ ID NO: 5).

In some embodiments, the oligonucleotide comprises or consists of a sequence complementary to the sequence of 5'-GCAAAGAATTCAGAAATT-3' (SEQ ID NO: 5).

In some embodiments, the oligonucleotide comprises or consists of:
a sequence having 70%, 75%, 80%, 85%, 90%, or 95% or more sequence identity to the sequence of 5'-ATTTCTGAATTCTTTGCC-3' (SEQ ID NO: 6) or 5'-AATTTCTGAATTCTTTGC-3' (SEQ ID NO: 7); or
the sequence of 5'-ATTTCTGAATTCTTTGCC-3' (SEQ ID NO: 6) or 5'-AATTTCTGAATTCTTTGC-3' (SEQ ID NO: 7), or a sequence that differs from the sequence of SEQ ID NO: 6 or SEQ ID NO: 7 in that 1 to 6, 1 to 5, 1 to 4, 1 to 3, 1 or 2 base(s) are modified in the sequence of SEQ ID NO: 6 or SEQ ID NO: 7.

In some embodiments, the oligonucleotide comprises or consists of the sequence of 5'-ATTTCTGAATTCTTTGCC-3' (SEQ ID NO: 6) or 5'-AATTTCTGAATTCTTTGC-3' (SEQ ID NO: 7).

In some embodiments, the oligonucleotide comprises or consists of the sequence of 5'-AATTTCTGAATTCTTTGC-3' (SEQ ID NO: 7).

As used herein, an oligonucleotide refers to a compound comprising a plurality of nucleosides linked together by inter-nucleoside bonds. A nucleoside refers to a compound comprising a sugar moiety and a base moiety. Nucleosides include ribonucleosides and deoxyribonucleosides. The oligonucleotide of the present disclosure may comprise any nucleoside and any inter-nucleoside bond known in the art and can be produced by known techniques for nucleic acid synthesis. In the oligonucleotide of the present disclosure, each nucleoside may be a naturally occurring or non-naturally occurring nucleoside, and each inter-nucleoside bond may be a naturally occurring or non-naturally occurring inter-nucleoside bond. An oligonucleotide comprising a non-naturally occurring nucleoside and/or a non-naturally occurring inter-nucleoside bond is also called a modified oligonucleotide.

A nucleoside can be a naturally occurring or non-naturally occurring nucleoside. A naturally occurring nucleoside refers to a compound comprising a naturally occurring sugar moiety and a naturally occurring base moiety, and a non-naturally occurring nucleoside refers to a compound comprising a non-naturally occurring sugar moiety and/or a non-naturally occurring base moiety.

The naturally occurring sugar moiety is ribose or 2'-deoxyribose of the furanose form. A non-naturally occurring sugar moiety may comprise modification of one or more atoms (for example, one or more positions selected from 2', 4', and 5' positions) of the ribofuranose ring; bridging of two atoms of the ribofuranose ring (bicyclic sugar); substitution of an oxygen atom (O) of the ribofuranose ring with a different atom (for example, S, N, or C), and substitution of the ribofuranose ring with a different structure; or any combination thereof.

Examples of modifications of the ribofuranose ring include halogen, amino, thio, alkyl, alkenyl, alkynyl, O-alkyl, O-alkenyl, O-alkynyl, S-alkyl, S-alkenyl, S-alkynyl, N-alkyl, N-alkenyl, N-alkynyl, allyl, O-allyl, S-allyl, N-allyl, and O-alkyl-O-alkyl, wherein the alkyl, alkenyl, alkynyl, and allyl may be substituted or unsubstituted alkyl, alkenyl, alkynyl, and allyl. In some embodiments, the modification of the ribofuranose ring is selected from 2'-F, 2'-OCH₃ (O-methyl), 2'-O(CH₂)₂OCH₃ (MOE), 4'-S, 5'-vinyl, and 5'-methyl.

Examples of nucleosides comprising a bicyclic sugar include nucleosides in which the atoms at 2' and 4' positions are bridged, such as LNA (locked Nucleic Acid/2'-O,4'-C-Methylene-bridged Nucleic Acid), AmNA (Amido-Bridged Nucleic Acid) and ENA (Ethylene-bridged Nucleic Acid).

A non-naturally occurring nucleoside may be a compound in which the sugar moiety is replaced by an acyclic structure, such as a Peptide Nucleic Acid (PNA).

The naturally occurring bases are adenine (A), thymine (T), cytosine (C), guanine (G), and uracil (U). Examples of non-naturally occurring bases include substituted forms of naturally occurring bases, such as 5-methylcytosine, 5-hydroxymethylcytosine, 6-methyladenine, 6-methylguanine, 4-thiouracil, and 5-fluorouracil. In the present disclosure, unless otherwise specified, an oligonucleotide sequence is represented by naturally occurring bases, but the oligonucleotide defined by such a sequence encompasses an oligonucleotide comprising one or more non-naturally occurring bases corresponding to the naturally occurring bases.

An inter-nucleoside bond can be a naturally occurring or non-naturally occurring inter-nucleoside bond. The naturally occurring inter-nucleoside bond is a phosphodiester bond. Examples of non-naturally occurring inter-nucleoside bonds include phosphotriester, phosphorothioate, boranophosphate, phosphorodithioate, alkylphosphonate, phosphoryl guanidine, methylphosphonate, methylene methylamino (-CH₂-N(CH₃)-O-CH₂-), thiodiester (-O-C(O)-S-), thionocarbamate (-O-C(O)(NH)-S-), siloxane (-O-Si(H)₂-O-), and N,N'-dimethylhydrazine (-CH₂-N(CH₃)-N(CH₃)-).

An oligonucleotide may comprise two or more types of nucleosides and/or two or more types of inter-nucleoside bonds. When an oligonucleotide comprises two or more types of nucleosides and/or two or more types of inter-nucleoside bonds, each of the nucleosides and inter-nucleoside bonds may be present at any proportion and arrangement in the oligonucleotide.

To make an oligonucleotide function as an SSO, types of nucleosides and/or inter-nucleoside bonds can be selected so as not to induce RNA degradation by RNase H. A person skilled in the art can appropriately select types and positions of nucleosides and inter-nucleoside bonds required to make an oligonucleotide function as an SSO. In some embodiments, an oligonucleotide does not comprise 6 or more, 5 or more, 4 or more, 3 or more, or 2 or more, preferably 6 or more, 5 or more, or 4 or more consecutive naturally occurring deoxyribonucleosides. A gapmer may be an oligonucleotide consisting of a middle region comprising 4 or more, 5 or more, or 6 or more consecutive naturally occurring deoxyribonucleosides and regions of 2 bases or more in length (for example, 2 to 10, 3 to 7, or 4 to 5 bases in length) located at the 5' and 3' ends of the middle region each comprising at least one non-naturally occurring nucleoside (referred to as a 5' wing region and a 3' wing region). Thus, in some embodiments, the oligonucleotide of the present disclosure is not an oligonucleotide consisting of a middle region comprising 4 or more, 5 or more, or 6 or more consecutive naturally occurring deoxyribonucleosides and regions of 2 bases or more in length (for example, 2 to 10, 3 to 7, or 4 to 5 bases in length) located at the 5' and 3' ends of the middle region each comprising at least one non-naturally occurring nucleoside.

An oligonucleotide may be 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 bases or more in length, and 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 bases or less in length, wherein the upper and lower limits can be independently selected. In some embodiments, the oligonucleotide is 13 to 23, 14 to 22, 15 to 21, or 16 to 20 bases in length, such as 16, 17, 18, 19, or 20 bases in length.

As used herein, a sequence "comprising" a predetermined base sequence encompasses a sequence that comprises the predetermined base sequence and one or more bases added thereto, and a sequence consisting of the predetermined base sequence.

With respect to base sequences, the sequence identity is the percentage of bases that match between two sequences that are optimally aligned (i.e., aligned to achieve maximum matches). The sequence identity is calculated by the following formula: sequence identity (%) = [(bases matched between two sequences)/(alignment length)] x 100. The sequence identity can be calculated by using programs such as FASTA and BLAST.

Base modification includes deletion, substitution, insertion, and addition of a base. The modification may be any one of deletion, substitution, insertion, and addition, or a combination of two or more of them. For example, when two or more bases are modified, each modification is independently selected from deletion, substitution, insertion, and addition.

In some embodiments, the oligonucleotide increases the amount of TAU mRNA including exon 1B by at least 10%, 20%, 30%, 40%, 50%, 60%, 70%, 80%, 90%, 100%, 150%, 200%, 250%, 300%, 350%, or 400% compared to the control.

In some embodiments, the oligonucleotide reduces the amount of TAU protein by at least 10%, 20%, 30%, 40%, 50%, 60%, 70%, 80%, or 90% compared to the control.

Whether an oligonucleotide can modulate splicing to increase the amount of TAU mRNA including exon 1B can be determined by quantitative PCR (qPCR) as described in Experiment 5 of the examples. qPCR uses a primer set of a forward primer designed to cover the junction between exon 1B and exon 4 and a reverse primer designed within exon 4 and another primer set of a forward primer designed to cover the junction between exon 1 and exon 4 and a reverse primer designed within exon 4. When the treatment with an oligonucleotide increases the amplification product with the former primer set and reduces the amplification product with the latter primer set, the oligonucleotide is determined to be capable of modulating splicing to increase the amount of TAU mRNA including exon 1B.

In some embodiments, the oligonucleotide increases the amount of TAU mRNA including exon 1B selectively in an excitatory neuron. In the present disclosure, an oligonucleotide is said to selectively increase the amount of TAU mRNA including exon 1B in an excitatory neuron when the increase in the amount of TAU mRNA including exon 1B induced by the oligonucleotide in an excitatory neuron is greater than that in an inhibitory neuron. Examples of excitatory neurons include glutamatergic neurons and examples of inhibitory neurons include GABAergic neurons. Whether an oligonucleotide can selectively increase the amount of TAU mRNA including exon 1B in an excitatory neuron can be determined, for example, by visualizing TAU mRNA in excitatory and inhibitory neurons by using a probe that can detect TAU mRNA including exon 1B, as described in Experiment 8 in the examples.

Whether an oligonucleotide can reduce the amount of TAU protein can be determined by usual quantitative methods that are thought to reflect the amount of protein, such as qPCR. In some embodiments, the oligonucleotide can selectively reduce the amount of TAU protein in an excitatory neuron. The selective reduction of the amount of TAU protein in an excitatory neuron can avoid side effects caused by unnecessary TAU suppression while suppressing neurodegeneration due to hyper synchronization of neural activity. In the present disclosure, an oligonucleotide is said to selectively reduce the amount of TAU protein in an excitatory neuron when the reduction in the amount of TAU protein induced by the oligonucleotide in an excitatory neuron is greater than that in an inhibitory neuron. Examples of excitatory neurons include glutamatergic neurons and examples of inhibitory neurons include GABAergic neurons. Whether an oligonucleotide can selectively reduce the amount of TAU protein in an excitatory neuron can be determined, for example, by measuring TAU mRNA expression, which is thought to correlate with the amount of TAU protein, by single cell RNA sequencing (scRNA-seq) and comparing the changes in the TAU mRNA expression between the oligonucleotide-treated groups in the excitatory and inhibitory neurons, as described in Experiment 9 in the examples.

In some embodiments, the increase in the amount of TAU mRNA including exon 1B or the reduction in the amount of TAU protein induced by an oligonucleotide in an excitatory neuron is at least 5%, 6%, 7%, 8%, 9%, 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, or 400% greater than the increase in the amount of TAU mRNA including exon 1B or the reduction in the amount of TAU protein induced by the oligonucleotide in an inhibitory neuron.

In some embodiments, the increase in the amount of TAU mRNA including exon 1B or the reduction in the amount of TAU protein in an excitatory neuron induced by an oligonucleotide is at least 1.5, 1.6, 1.7, 1.8, 1.9, 2.0, 2.1, 2.2, 2.3, 2.4, 2.5, 2.6, 2.7, 2.8, 2.9, or 3.0 times greater than the increase in the amount of TAU mRNA including exon 1B or the reduction in the amount of TAU protein induced by the oligonucleotide in an inhibitory neuron.

In some embodiments, the oligonucleotide is selected from the followings.

| Sequence (5'→3') | SEQ ID NO: |
|---|---|
| A_{Ls}T_{ds}T_{Ls}T_{ds}^{m}C_{Ls}T_{ds}G_{Ls}A_{ds}A_{Ls}T_{ds}T_{Ls}^{m}C_{ds}T_{Ls}T_{ds}T_{Ls}G_{ds}^{m}C_{Ls}^{m}C_{d} | 12 |
| A_{Ls}A_{ds}T_{Ls}T_{ds}T_{Ls}^{m}C_{ds}T_{Ls}G_{ds}A_{Ls}A_{ds}T_{Ls}T_{ds}C_{Ls}T_{ds}T_{Ls}T_{ds}G_{Ls}^{m}C_{d} | 13 |
| A_{As}T_{dn}T_{As}T_{dn}^{m}C_{As}TₐₛG_{As}A_{ds}A_{As}T_{ds}T_{As}^{m}C_{ds}T_{As}T_{dn}T_{As}G_{dn}^{m}C_{As}^{m}C_{d} | 16 |
| A_{As}A_{dn}T_{As}T_{dn}T_{As}^{m}C_{ds}T_{As}G_{ds}A_{As}A_{ds}T_{As}T_{ds}^{m}C_{As}T_{dn}T_{As}T_{dn}G_{As}^{m}C_{d} | 17 |

| | |
|---|---|
| "d" = DNA, "L" = LNA, "A" = AmNA, "s" = phosphorothioate, "n" = phosphoramidate, "^{m}C" = 5-methylcytosine. | |

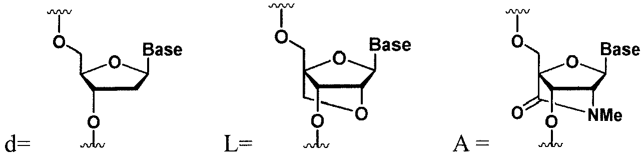

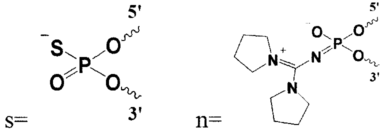

The oligonucleotide of the present disclosure can reduce the amount of TAU protein and can be used to treat or prevent a TAU-related disease. As used herein, a TAU-related disease refers to a disease or condition in which accumulation or heterologous localization of TAU is involved in its onset or progression.

Examples of TAU-related diseases include tauopathy and epilepsy. Further examples of TAU-related diseases include dementia, such as Alzheimer's dementia (AD), vascular dementia, and dementia with Lewy bodies (DLB), Parkinson's disease, amyotrophic lateral sclerosis (ALS), Down syndrome (Down), Pick's disease (Pick), chronic traumatic encephalopathy (CTE), progressive supranuclear palsy (PSP), corticobasal degeneration (CBD), and myotonic dystrophy type 1 (DM1). Further examples of TAU-related diseases include Dravet syndrome, tuberous sclerosis complex (TSC), episodic ataxia (EA) (such as EA-1 or EA-2), cortical dysplasia-focal epilepsy (CDFE) syndrome, and developmental and epileptic encephalopathy (DEE) 14 or 18. Further examples of TAU-related diseases include ophthalmic diseases such as glaucoma and age-related macular degeneration (AMD).

The treatment of a TAU-related disease includes improvement and suppression of progression of one or more symptoms or findings of the disease. The prevention of a TAU-related disease includes suppression and delay of the onset of the disease.

The oligonucleotide is administered to a subject in an amount that can produce a desired effect (for example, effectiveness of treatment or prevention of a TAU-related disease) (herein also referred to as an effective amount). The dosage is appropriately selected according to factors such as age, body weight, and health condition of the subject. The oligonucleotide may be administered, for example, at a dose of 0.01 mg to 10 mg, 0.01 mg to 5 mg, 0.01 mg to 1 mg, 0.01 mg to 0.5 mg, or 0.05 mg to 0.5 mg per kg body weight. The oligonucleotide may be administered multiple times, and may be administered for consecutive days, daily or once every several days, weekly or once every several weeks, monthly or once every several months, once every several years or once in a lifetime. The dosage may be changed during the dosing period. The daily dose may be administered as a single dose or divided into multiple doses (for example, two to four doses).

The mode of administration is appropriately selected according to factors such as age, body weight, and health condition of the subject. The mode of administration may be oral or parenteral, but preferably parenteral. Examples of parenteral administration includes subcutaneous, intradermal, intramuscular, intraperitoneal, intravenous, intrathecal, subarachnoid, intra-cerebral parenchymal, intra-cerebral ventricular, intravitreal, suprachoroidal, or subretinal administration. The oligonucleotide may be administered, for example, by injection.

In addition to the oligonucleotide, a pharmaceutical composition may comprise a pharmaceutically acceptable carrier or additive, such as sterile water, saline, stabilizer, excipient, antioxidant, buffer, preservative, surfactant, chelating agent, or binder. The oligonucleotide may be encapsulated in a carrier suitable for administration of nucleic acid medicines such as a liposome. The dosage form is not limited to, but may be, an injection. Examples of injections include solution injection, suspension injection, emulsion injection, and an injection to be prepared before using (for example, lyophilized injection). The pharmaceutical composition may be provided as a kit, and the kit may further comprise, for example, a buffer solution to be used for preparation before using or an instruction for use.

The subject may be a mammal, for example, human or non-human mammal. Examples of non-human mammals include, for example, mouse, rat, rabbit, cat, dog, sheep, pig, horse, cattle, and monkey. In some embodiments, the subject is a human.

In one aspect, the present disclosure relates to a method for treating or preventing a TAU-related disease, comprising administering to a subject an effective amount of the splice-switching oligonucleotide that binds to intron 1B of TAU pre-mRNA.

In one aspect, the present disclosure relates to a splice-switching oligonucleotide that binds to intron 1B of TAU pre-mRNA for use in the treatment or prevention of a TAU-related disease.

In one aspect, the present disclosure relates to use of a splice-switching oligonucleotide that binds to intron 1B of TAU pre-mRNA for the manufacture of a medicament for treating or preventing a TAU-related disease.

Exemplary embodiments of the present disclosure are described below.
1. A splice-switching oligonucleotide that binds to intron 1B of TAU pre-mRNA.
2. The splice-switching oligonucleotide according to item 1, which is capable of increasing an amount of TAU mRNA including TAU exon 1B.
3. The splice-switching oligonucleotide according to item 1 or 2, which is capable of reducing an amount of TAU protein.
4. The splice-switching oligonucleotide according to any one of items 1 to 3, which comprises a sequence complementary to a part of intron 1B of TAU pre-mRNA.
5. The splice-switching oligonucleotide according to any one of items 1 to 4, which comprises a sequence complementary to at least a part of the sequence at positions 6 to 24 of SEQ ID NO: 2.
6. The splice-switching oligonucleotide according to any one of items 1 to 5, which comprises:
   a sequence complementary to a sequence having 70%, 75%, 80%, 85%, 90%, or 95% or more sequence identity to the sequence of 5'-GGCAAAGAATTCAGAAATT-3' (SEQ ID NO: 3); or
   a sequence complementary to the sequence of 5'-GGCAAAGAATTCAGAAATT-3' (SEQ ID NO: 3), or a sequence complementary to a sequence that differs from the sequence of SEQ ID NO: 3 in that 1 to 6, 1 to 5, 1 to 4, 1 to 3, 1 or 2 base(s) are modified in the sequence of SEQ ID NO: 3.
7. The splice-switching oligonucleotide according to any one of items 1 to 6, which comprises a sequence complementary to a sequence consisting of at least 13 consecutive bases in the sequence of 5'-GGCAAAGAATTCAGAAATT-3' (SEQ ID NO: 3).
8. The splice-switching oligonucleotide according to any one of items 1 to 7, which comprises or consists of:
   a sequence complementary to a sequence having 70%, 75%, 80%, 85%, 90%, or 95% or more sequence identity to the sequence of 5'-GGCAAAGAATTCAGAAAT-3' (SEQ ID NO: 4) or 5'-GCAAAGAATTCAGAAATT3' (SEQ ID NO: 5); or
   a sequence complementary to the sequence of 5'-GGCAAAGAATTCAGAAAT-3' (SEQ ID NO: 4) or 5'-GCAAAGAATTCAGAAATT-3' (SEQ ID NO: 5), or a sequence complementary to a sequence that differs from the sequence of SEQ ID NO: 4 or SEQ ID NO: 5 in that 1 to 6, 1 to 5, 1 to 4, 1 to 3, 1 or 2 base(s) are modified in the sequence of SEQ ID NO: 4 or SEQ ID NO: 5.
9. The splice-switching oligonucleotide according to any one of items 1 to 8, which comprises or consists of a sequence complementary to the sequence of 5'-GGCAAAGAATTCAGAAAT-3' (SEQ ID NO: 4) or 5'-GCAAAGAATTCAGAAATT-3' (SEQ ID NO: 5).
10. The splice-switching oligonucleotide according to any one of items 1 to 9, which comprises or consists of:
   a sequence having 70%, 75%, 80%, 85%, 90%, or 95% or more sequence identity to the sequence of 5'-ATTTCTGAATTCTTTGCC-3' (SEQ ID NO: 6) or 5'-AATTTCTGAATTCTTTGC-3' (SEQ ID NO: 7); or
   the sequence of 5'-ATTTCTGAATTCTTTGCC-3' (SEQ ID NO: 6) or 5'-AATTTCTGAATTCTTTGC-3' (SEQ ID NO: 7), or a sequence that differs from the sequence of SEQ ID NO: 6 or SEQ ID NO: 7 in that 1 to 6, 1 to 5, 1 to 4, 1 to 3, 1 or 2 base(s) are modified in the sequence of SEQ ID NO: 6 or SEQ ID NO: 7.
11. The splice-switching oligonucleotide according to any one of items 1 to 10, which comprises or consists of the sequence of 5'-ATTTCTGAATTCTTTGCC-3' (SEQ ID NO: 6) or 5'-AATTTCTGAATTCTTTGC-3' (SEQ ID NO: 7).
12. The splice-switching oligonucleotide according to any one of items 1 to 11, which does not comprise 6 or more, 5 or more, or 4 or more consecutive naturally occurring deoxyribonucleosides.
13. The splice-switching oligonucleotide according to any one of items 1 to 12, which is not an oligonucleotide consisting of a middle region comprising 4 or more, 5 or more, or 6 or more consecutive naturally occurring deoxyribonucleosides and regions of 2 bases or more in length located at the 5' and 3' ends of the middle region each comprising at least one non-naturally occurring nucleoside.
14. The splice-switching oligonucleotide according to any one of items 1 to 13, which is 16 to 20 bases in length.
15. The splice-switching oligonucleotide according to any one of items 1 to 14, which is capable of increasing an amount of TAU mRNA including TAU exon 1B selectively in an excitatory neuron.
16. The splice-switching oligonucleotide according to any one of items 1 to 15, which is capable of increasing an amount of TAU mRNA including TAU exon 1B in an excitatory neuron by at least 5% greater than in an inhibitory neuron.
17. The splice-switching oligonucleotide according to any one of items 1 to 16, which is capable of reducing an amount of TAU protein selectively in an excitatory neuron.
18. The splice-switching oligonucleotide according to any one of items 1 to 17, which is capable of reducing an amount of TAU protein in an excitatory neuron by at least 5% greater than in an inhibitory neuron.
19. An oligonucleotide, comprising or consisting of:
   a sequence complementary to a sequence having 70%, 75%, 80%, 85%, 90%, or 95% or more sequence identity to the sequence of 5'-GGCAAAGAATTCAGAAAT-3' (SEQ ID NO: 4) or 5'-GCAAAGAATTCAGAAATT-3' (SEQ ID NO: 5); or
   a sequence complementary to the sequence of 5'-GGCAAAGAATTCAGAAAT-3' (SEQ ID NO: 4) or 5'-GCAAAGAATTCAGAAATT-3' (SEQ ID NO: 5), or a sequence complementary to a sequence that differs from the sequence of SEQ ID NO: 4 or SEQ ID NO: 5 in that 1 to 6, 1 to 5, 1 to 4, 1 to 3, 1 or 2 base(s) are modified in the sequence of SEQ ID NO: 4 or SEQ ID NO: 5.
20. An oligonucleotide, comprising or consisting of:
   a sequence having 70%, 75%, 80%, 85%, 90%, or 95% or more sequence identity to the sequence of 5'-ATTTCTGAATTCTTTGCC-3' (SEQ ID NO: 6) or 5'-AATTTCTGAATTCTTTGC-3' (SEQ ID NO: 7); or
   the sequence of 5'-ATTTCTGAATTCTTTGCC-3' (SEQ ID NO: 6) or 5'-AATTTCTGAATTCTTTGC-3' (SEQ ID NO: 7), or a sequence that differs from the sequence of SEQ ID NO: 6 or SEQ ID NO: 7 in that 1 to 6, 1 to 5, 1 to 4, 1 to 3, 1 or 2 base(s) are modified in the sequence of SEQ ID NO: 6 or SEQ ID NO: 7.
21. A pharmaceutical composition comprising the splice-switching oligonucleotide according to any one of items 1 to 18, or the oligonucleotide according to item 17 or 18.
22. The pharmaceutical composition according to item 21, which is for treating or preventing a TAU-related disease.
23. The pharmaceutical composition according to item 22, wherein the TAU-related disease is tauopathy or epilepsy.
24. The pharmaceutical composition according to item 22, wherein the TAU-related disease is selected from the group consisting of Alzheimer's dementia, vascular dementia, dementia with Lewy bodies, Parkinson's disease, amyotrophic lateral sclerosis, Down syndrome, Pick's disease, chronic traumatic encephalopathy, progressive supranuclear palsy, corticobasal degeneration, and myotonic dystrophy type 1.
25. The pharmaceutical composition according to item 22, wherein the TAU-related disease is selected from the group consisting of Dravet syndrome, tuberous sclerosis complex, episodic ataxia, CDFE syndrome, and DEE14 or 18.
26. The pharmaceutical composition according to item 22, wherein the TAU-related disease is selected from the group consisting of glaucoma and age-related macular degeneration.
27. The pharmaceutical composition according to item 21, which is for attenuating neurodegeneration due to hyper synchronization of neural activity.
28. A method for treating or preventing a TAU-related disease, comprising administering to a subject an effective amount of the splice-switching oligonucleotide according to any one of items 1 to 18, the oligonucleotide according to item 19 or 21, or the pharmaceutical composition according to any one of items 21 to 27.
29. The splice-switching oligonucleotide according to any one of items 1 to 18 or the oligonucleotide according to item 19 or 20 for use in treating or preventing a TAU-related disease.
30. Use of the splice-switching oligonucleotide according to any one of items 1 to 18 or the oligonucleotide according to item 19 or 20 for the manufacture of a medicament for treating or preventing a TAU-related disease.

### Examples

### 1. Synthesis and purification of oligonucleotides

Oligonucleotides shown in Table 1 were synthesized.

**Table 1**

| Compound No. | Sequence (5'→3') | SEQ ID NO: |
|---|---|---|
| Scramble | GₘₛGₘₒGₘₛ^{m}CₘₛGₘₛTₘₛA_{ds}T_{ds}A_{ds}G_{ds}A_{ds}^{m}C_{ds}G_{ds}TₘₛGₘₒTₘₛTₘₛAₘ | 8 |
| Comparative example compound 1 | ^{m}Cₘₛ^{m}CₘₒGₘₛTₘₛTₘₛT_{ds}T_{ds}^{m}C_{ds}T_{ds}T_{ds}A_{ds}^{m}C_{ds}^{m}C_{ds}Aₘₛ^{m}Cₘₒ^{m}Cₘₛ^{m}CₘₛTₘ | 9 |
| **1** | T_{Ls}T_{ds}^{m}C_{Ls}T_{ds}G_{Ls}A_{ds}A_{Ls}T_{ds}T_{Ls}^{m}C_{ds}T_{Ls}T_{ds}T_{Ls}G_{ds}^{m}C_{Ls}^{m}C_{ds}A_{Ls}G_{d} | 10 |
| **2** | T_{Ls}T_{ds}T_{Ls}^{m}C_{ds}T_{Ls}G_{ds}A_{Ls}A_{ds}T_{Ls}T_{ds}^{m}C_{Ls}T_{ds}T_{Ls}T_{ds}G_{Ls}^{m}C_{ds}^{m}C_{Ls}A_{d} | 11 |
| **3** | A_{Ls}T_{ds}T_{Ls}T_{ds}^{m}C_{Ls}T_{ds}G_{Ls}A_{ds}A_{Ls}T_{ds}T_{Ls}^{m}C_{ds}T_{Ls}T_{ds}T_{Ls}G_{ds}^{m}C_{Ls}^{m}C_{d} | 12 |
| **4** | A_{Ls}A_{ds}T_{Ls}T_{ds}T_{Ls}^{m}C_{ds}T_{Ls}G_{ds}A_{Ls}A_{ds}T_{Ls}T_{dS}^{m}C_{Ls}T_{ds}T_{Ls}T_{ds}G_{Ls}^{m}C_{d} | 13 |
| **5** | G_{Ls}A_{ds}A_{Ls}T_{ds}T_{Ls}T_{ds}^{m}C_{Ls}T_{ds}G_{Ls}A_{ds}A_{Ls}T_{ds}T_{Ls}^{m}C_{ds}T_{Ls}T_{ds}T_{Ls}G_{d} | 14 |
| **6** | A_{Ls}G_{ds}A_{Ls}A_{ds}T_{Ls}T_{ds}T_{Ls}^{m}C_{ds}T_{Ls}G_{ds}A_{Ls}A_{ds}T_{Ls}T_{ds}^{m}C_{Ls}T_{ds}T_{Ls}T_{d} | 15 |
| **7** | A_{As}T_{dn}T_{As}T_{dn}^{m}C_{As}T_{ds}G_{As}A_{ds}A_{As}T_{ds}T_{As}^{m}C_{ds}T_{As}T_{dn}T_{As}G_{dn}^{m}C_{As}^{m}C_{d} | 16 |
| **8** | A_{As}A_{dn}T_{As}T_{dn}T_{As}^{m}C_{ds}T_{As}G_{ds}A_{As}A_{ds}T_{As}T_{ds}^{m}C_{As}T_{dn}T_{As}T_{dn}G_{As}^{m}C_{d} | 17 |

| | | |
|---|---|---|
| "d" = DNA, "m" (subscript) = 2'-MOE, "L" = LNA, "A" = AmNA, "o" = phosphodiester, "s" = phosphorothioate, "n" = phosphoramidate, "^{m}C" = 5-methylcytosine. | | |

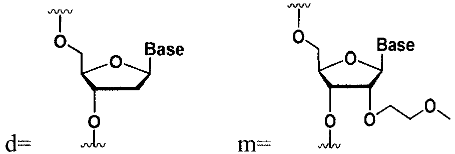

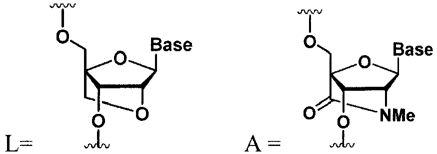

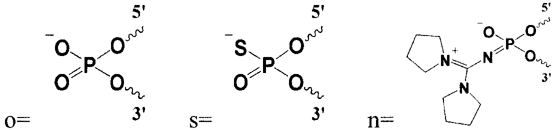

### <Methods>

The synthesis of compounds 1, 2, 3, 4, 5, 6, 7, and 8 (Table 1) was performed according to the procedure shown in Table 2. A commercially available 5'-O-DMTr nucleoside CPG (1 µmol scale) was used in an automated nucleic acid synthesizer (M-8-MX, Nippon Techno Service) to elongate the chain by repeating steps 1 to 8 of Table 2 and then the 5'-O-DMTr group was removed with 3% DCA in CH₂Cl₂. Deprotection of protecting groups of nucleobases and phosphate groups and cleavage of linkers for compounds 1, 2, 3, 4, 5, and 6 were accomplished by treatment with 25% NH₃ aqueous solution for 5 hours at 55°C. Deprotection of protecting groups of nucleobases and phosphate groups and cleavage of linkers of compounds 7 and 8 were accomplished by treatment with 20% diethylamine in MeCN for 1 h at 25°C followed by treatment with a mixture of TEA-MeOH-water (1:1:2, v/v/v) at 65°C for 20 hours. The crude product obtained was purified by ion-exchange HPLC (DNAPac PA100, Thermo Scientific). Fractions containing the oligonucleotides of interest were collected, purified by a simple reversed-phase column (Sep-Pak, Waters), and lyophilized. The yield was determined by UV quantification at 260 nm (Shimazu UV-1800, Shimadzu Corporation) and its composition was identified by LC-MS (Xevo G2-XS Qtof, Waters).

### <Results>

Compound 1: Yield 1.2 mg, ESI-MS: m/z calcd 6031.6, found 6030.7
Compound 2: Yield 1.1 mg, ESI-MS: m/z calcd 6006.5, found 6005.7
Compound 3: Yield 0.2 mg, ESI-MS: m/z calcd 6006.5, found 6006.0
Compound 4: Yield 1.0 mg, ESI-MS: m/z calcd 6016.5, found 6015.7
Compound 5: Yield 0.7 mg, ESI-MS: m/z calcd 6042.5, found 6041.7
Compound 6: Yield 1.4 mg, ESI-MS: m/z calcd 6026.5, found 6025.9
Compound 7: Yield 0.8 mg, ESI-MS: m/z calcd 6768.2, found 6768.3
Compound 8: Yield 2.6 mg, ESI-MS: m/z calcd 6775.3, found 6777.9

**Table 2**

| step | operation | synthetic cycle for phosphorothioate | | synthetic cycle for phosphoramidate | |
|---|---|---|---|---|---|
| | | reagent | time | reagent | time |
| 1 | detritylation | 3% TCA in CH₂Cl₂ | 7s | 3% TCA in CH₂Cl₂ | 7s |
| 2 | washing | CH₃CN | - | CH₃CN | - |
| 3 | condensation | 0.07 M monomer and 0.25 M BTT in CH₃CN | 9min | 0.07 M monomer and 0.25 M BTT in CH₃CN | 9min |
| 4 | washing | CH₃CN | - | CH₃CN | - |
| 5 | capping | Ac₂O and 16% NMI in THF | 40s | Ac₂O and 16% NMI in THF | 40s |
| 6 | washing | CH₃CN | - | CH₃CN | - |
| 7 | sulfurization/ imidation | 0.05 M POS in CH₃CN | 8min | 1 M 1-(azide-1-pyrrolidinyl-methylene)pyrrolidin ium hexafluorophosphate in CH₃CN | 30min |
| 8 | washing | CH₃CN | - | CH₃CN | - |

The compound Scramble and comparative example compound 1 were synthesized in the same way. The compound Scramble is a negative control. Comparative example compound 1 is an example of a gapmer-type antisense oligonucleotide against TAU mRNA.

### 2. Identification of sense sequence which is used to promote inclusion of exon 1B

To identify SSO-type antisense oligonucleotides, cDNA from human iPS cell-derived neuronal cells (hereinafter referred to as "iPS neurons") treated with compound 2, 3, 4, or 5 was subjected to RT-PCR and electrophoresis and the splice-switching activity was qualitatively evaluated.

### <Methods>

iCell GlutaNeurons 01279 (Fujifilm Cellular Dynamics, Inc.), a product with a mixture of glutamatergic (excitatory) and GABAergic (inhibitory) neurons, was used according to the user's instructions. Specifically, a microplate Corning^{®} BioCoat^{™} poly-D-lysine 384-well Black/Clear Flat Bottom TC-treated Microplate (Corning) was coated with poly-L-ornithine (sigma-Aldrich) and Corning^{®} Matrigel Growth Factor Reduced Basement Membrane Matrix (Corning). Then iPS neurons were suspended in Complete BrainPhys medium (without added antibiotics) (hereinafter referred to as "the medium") and seeded on the microplate (26,000 to 30,000 cells/well, final volume of 40 µL/well). On the next day, to prepare the final concentration of the oligonucleotide in the culture medium 1 µM, an equal volume of the medium containing 2 µM oligonucleotide, the concentration of which was double of the final concentration, was added to the cells (final volume of 80 µL/well). To cultivate cells without the oligonucleotide treatment, distilled water (hereafter referred to as d.w., Otsuka Pharmaceutical Factory, Inc.) was added at 0.1 %. For medium change, 40 µL of the culture supernatant was discarded and a fresh 40 µL of the medium containing 1 µM oligonucleotide was added every 3 days.

To collect a lysate on day 16 after seeding, the whole of the medium was removed and 7 µL of RIPA Buffer (1x ready-to-use solution, protease inhibitor free) (Nacalai Tesque) was added to lyse the cells by agitation followed by freeze-thawing (the resulting lysate was called RIPA lysate). Next, cDNA was synthesized by the following procedure. According to the user's instructions of SuperPrep^{®} II Cell Lysis & RT Kit for qPCR (TOYOBO), 9 µL of a lysis buffer (a mixture of 8.75 µL of Lysis Solution, 0.2 µL of RNase Inhibitor, and 0.05 µL of gDNA Remover) and 1 µL of the RIPA lysate was mixed and the mixture was left to stand at room temperature for 5 minutes to degrade the genomic DNA and obtain an RNA-containing solution. Then, for reverse transcription, 5X RT Master Mix, the RNA-containing solution and d.w. were mixed and reacted at 37°C (15 min), 50°C (5 min) and 98°C (5 min) to obtain a cDNA solution. The cDNA solution was then diluted 10-fold with d.w.

To perform PCR, TAU primers were added to PrimeSTAR^{®} GXL Premix (TAKARA) at a final concentration of 200nM, or β-actin (ACTB) primers were added to the same at a final concentration of 100nM, after the primers were diluted with d.w. The following primers were synthesized and used (Integrated DNA Technologies, Inc./IDT).
<For TAU mRNA detection>
   118 (118-Fw-Mapt-Exon1) (5'-CATGCACCAAGACCAAGAGG-3') (SEQ ID NO: 18),
   120 (120-Rv-Mapt-Exon4) (5'-TCACGTGACCAGCAGCTTC-3') (SEQ ID NO: 19).
<For ACTB mRNA detection>
   (5'-ACAGAGCCTCGCCTTTG-3') (SEQ ID NO: 20), (5'-CCTTGCACATGCCGGAG-3') (SEQ ID NO: 21), primers in Hs.PT.39a.22214847 of IDT.

Each synthetic DNA template for a positive control of RT-PCR was synthesized with the following sequence (gBLOCKS, IDT).
110-TAU nonproductive mRNA (DNA-standard) (E1B-E4): possessing exon 1B sequence between exon 1 and exon 4. 
110 (5'-TCAGGGGGGCTACACCATGCACCAAGACCAAGAGGGTGACACGGAC
GCTGGCCTGAAAGAGGGGAGTGAATTCACTGAGCTGAGAGCTGAGGAACCA
TTGATCTGATGGCTGAGACACCACTGGGAAGACTGGAGAGGCTTTTCTGGGC
ATGCAGTGCCAGGCACAGGAGGAGCTGAGGGAAGATGACTAAGAGCTGAAG
AAGCAGGCATTGGAGACACCCCCAGCCTGGAAGACGAAGCTGCTGGTCACG
TGACCCAA-3') (SEQ ID NO: 22)
111-TAU productive mRNA (DNA-standard) (E1-E4): possessing exon 1 followed by a sequence of exon 4.

111 (5'-TCAGGGGGGCTACACCATGCACCAAGACCAAGAGGGTGACACGGAC
GCTGGCCTGAAAGCTGAAGAAGCAGGCATTGGAGACACCCCCAGCCTGGAA
GACGAAGCTGCTGGTCACGTGACCCAAG-3') (SEQ DI NO: 23)

ACTB (5'-GCATCGACATCGACTAACCGCCGAGACCGCGTCCGCCCCGCGAGC
ACAGAGCCTCGCCTTTGCCGATCCGCCGCCCGTCCACACCCGCCGCCAGCTC
ACCATGGATGATGATATCGCCGCGCTCGTCGTCGACAACGGCTCCGGCATGTG
CAAGGCCGGCTTCGCGGGCGACGATGCCCCCCGGGCCGTCTTCCCCTCCATC
GTGGGGCGCCCCAGGCACCAGGGCGTGATGGTGGGCATGGGTCAGAAGGAT
TCCTATGTGGGCGACGAGGCCCAGAGCAAGAGAGGCATCCTCACCCTGAAG
TACCCCATCGAGCACGGCATCGTCACCAACTGGGACGACATGGAGAAAATCT
GGCACCACAC-3') (SEQ ID NO: 24)

The touchdown PCR was performed with the thermal cycler GeneAmp PCR System 9700 (Applied Biosystems) and the reaction comprised, in this order, 3 cycles [denaturation: 98°C (10 sec); 66°C (15 sec); 68°C (15 sec)], 3 cycles [denaturation: 98°C (10 sec); 64°C (15 sec); 68°C (15 sec)], 10 cycles [denaturation: 98°C (10 sec); 62°C (15 sec); 68°C (15 sec)], and 40 cycles [denaturation: 98°C (10 sec); 60°C (15 sec); 68°C (15 sec)]. Then, 5 µL of the amplified PCR product was mixed with 1 µL of Gel Loading Dye, Purple (6X) (New England Biolabs Japan, Inc) and electrophoresed on 3% agarose 21 (for separation of low molecular weight nucleic acids) (FUJIFILM Wako Pure Chemical Corporation) using TAE (Nippon Gene). 100bp DNA Ladder One (Nacalai Tesque) was used as a marker. To detect the bands, gels were stained with SYBR^{™} Gold Nucleic Acid Gel Stain (10,000X Concentrate in DMSO) (Thermo Fisher Scientific K.K.) diluted 10,000-fold with TAE under light-shielded condition and visualized for fluorescence (Cy2) with Amersham^{™} ImageQuant^{™} 800 series (IQ800) (Global Life Sciences Technologies Japan K.K).

### <Results>

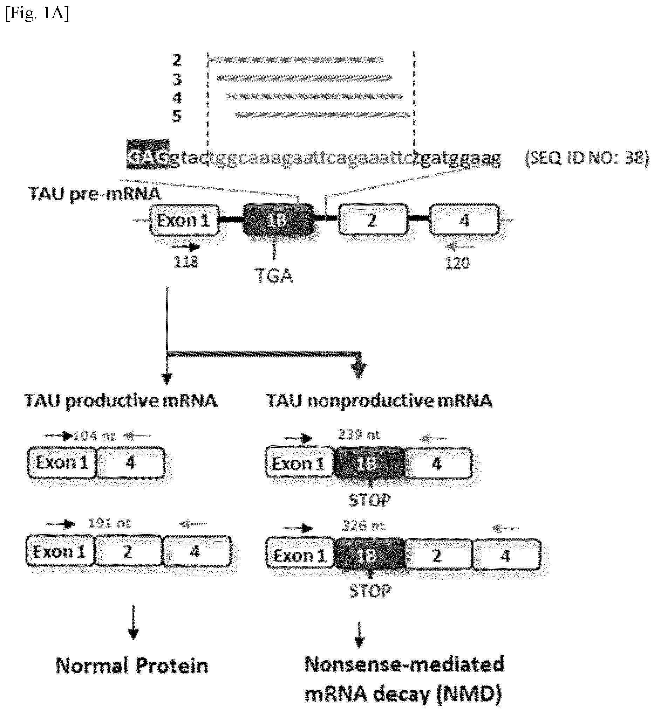

The 135 nucleotides (nt) that have been recognized as a part of introns was registered as an exon in ENST00000571311.5. The 135 nucleotides were included between exon 1 and exon 2 (Fig. 1A). This 135 nt exon is hereinafter referred to as exon 1B. Since a premature termination codon appears due to the inclusion of exon 1B, this transcript is assumed to be degraded via NMD (nonsense-mediated mRNA decay) and therefore referred to as nonproductive mRNA. In humans, for productive mRNA, which is the basis of TAU protein, three variants of N-terminal region had been reported: 1N TAU, which includes exon 2 but not exon 3; 2N TAU, which includes exons 2 and 3; and 0N TAU, which include neither exon 2 nor 3 (Wang JZ et al., Prog Neurobiol. 85. 148-75. 2008, Fig. 5). Therefore, there were three possible types for nonproductive mRNA including exon 1B. In this study, however, two nonproductive mRNA expression patterns were mainly detected by RT-PCR in iPS neurons. Fig. 1A shows only the transcripts actually detected in this study.

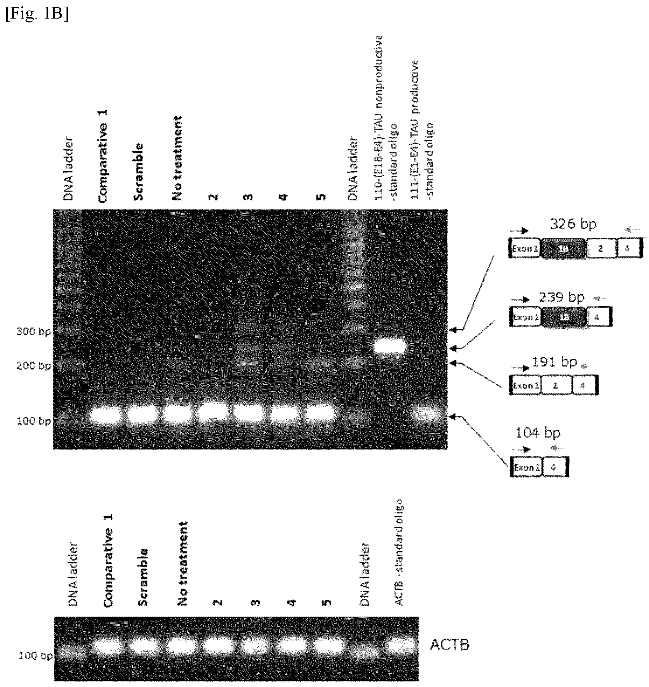

To identify a sense sequence on the pre-mRNA side that promotes inclusion of exon 1B by an SSO and increases nonproductive mRNA, oligonucleotides consisting of 18 bases were introduced into iPS neurons at 1 µM without any transfection reagent (i.e., by free uptake) and then mRNA variants (transcripts) of TAU were evaluated. To characterize the transcripts, a forward primer 118 and a reverse primer 120 were designed for exon 1 and exon 4, respectively, and differences in molecular weight of RT-PCR products were assessed. Without the oligonucleotide treatment, bands were detected at around 100 base pairs (bp) and 200bp (Fig. 1B, top). The band observed at around 100bp was detected at the same molecular weight as the synthetic DNA template of the 0N TAU positive control, suggesting that it is a productive mRNA, in which exon 1 was followed by exon 4 and joined to it. The band at around 200bp was considered to possess a molecular weight equivalent to 1N TAU. With the oligonucleotide treatment, compound 3 or 4 resulted in detection of each novel band in which 135bp was added to each of the bands at around 100bp and 200bp described above (Fig. 1B, top). The band at around 235bp was considered to be a nonproductive mRNA because it had the same molecular weight as the synthetic DNA template of the positive control, in which exon 1B of 135bp was included in 0N TAU of 104bp. The band observed at around 335bp was considered to be a nonproductive mRNA with the 135bp inclusion in 1N TAU of 191bp. β-actin was used as a loading control to display the presence of cDNA other than TAU (Fig. 1B, bottom).

### 3. Confirmation of exon structure by Sanger sequencing

To determine to which exon-exon junction the 135nt exon 1B was included, bands at around 235bp and 335bp found in the compound 4-treated sample were extracted, cloned, and analyzed by Sanger sequencing.

### <Methods>

cDNA from the compound 4-treated sample was obtained as described in Experiment 2 above and PCR products from the cDNA with the primers 118 and 120 were electrophoresed. Bands at around 335bp, 235bp, and 200bp were extracted from the gel and purified with the GEL/PCR Purification Mini Kit (FAVORGEN) according to the manual. To add dA by using 10x A-attachment Mix (TOYOBO), 0.5 µL of 10x A-attachment Mix was added to 4.5 µL of the purified sample and allowed to react at 60°C for 30 minutes. For cloning into pT7Blue T-vector (Novagen), 2 µL of each sample was added to 0.5 µL of pT7Blue T-Vector, and to the obtained mixture, 2.5 µL of DNA Ligation Kit <Mighty Mix> (TAKARA) was added. The sample was reacted at 16°C for 30 minutes. The entire solution was used for transformation of ready-to-use competent cells (E. coli JM109 strain) by heat treatment (42°C, 30 seconds) and the cells were plated on ampicillin-containing LB agar plates supplemented with X-gal and IPTG (Unitech Inc., Cat. No. LBA-Op). After overnight incubation at 37°C, white or light blue colonies were picked up and incubated in 100 µL of LB medium containing ampicillin (final concentration of 100 µg/mL, Unitech Inc.). Once the LB medium started to be cloudy, a solution for PCR reaction was prepared by mixing 2 µL of the LB medium with PrimeSTAR GXL DNA Polymerase (TAKARA) and primers (final concentration of 0.2 or 0.3 µM) (the reaction scale was 15 µL). The following primers were used: pT7Blue-F-m13r (5'-CAGGCTTTACACTTTATGCTTCC-3') (SEQ ID NO: 25); pT7Blue-R2-u19 (5'-CGATTTCGGCCTATTGGTTA-3') (SEQ ID NO: 26). PCR was performed with a thermal cycler GeneAmp PCR System 9700 (Applied Biosystems) with 30 cycles [denaturation: 98°C (10 sec); 60°C (15 sec); 68°C (30 sec)]. The band size was confirmed by electrophoresis of the colony PCR sample (5 µL) of each clone on an agarose gel (2%). Selected clones were cultured in an ampicillin-containing LB medium in 1 mL scale and plasmids were extracted by a usual alkaline-SDS lysis. For sequencing analysis, BigDye Terminator v3.1 Cycle sequencing Kit (Applied Biosystems) was used to sequence the purified plasmid (template) with either of the above primers (pT7Blue-F-m13r or pT7Blue-R2-u19) by paired-end sequencing. The sequence reaction was performed for 25 cycles [denaturation: 96°C (10 sec); 50°C (5 sec); 60°C (2 min)]. ABI PRISM 3130xl Genetic Analyzer (Applied Biosystems) was used for the measurements.

### <Results>

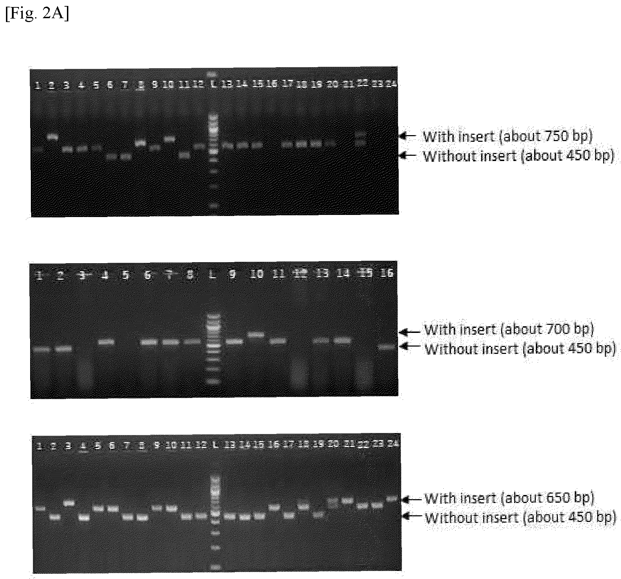

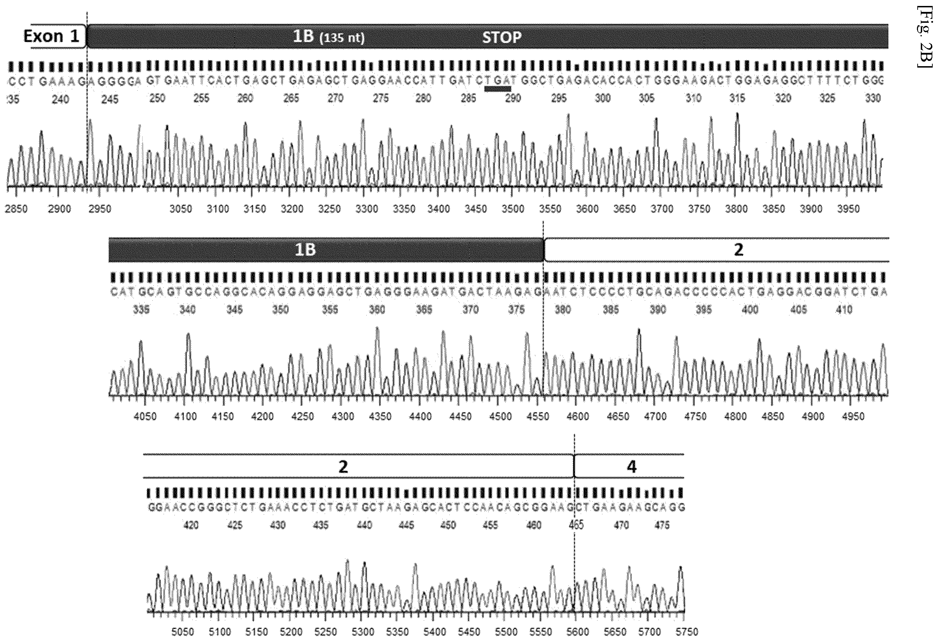

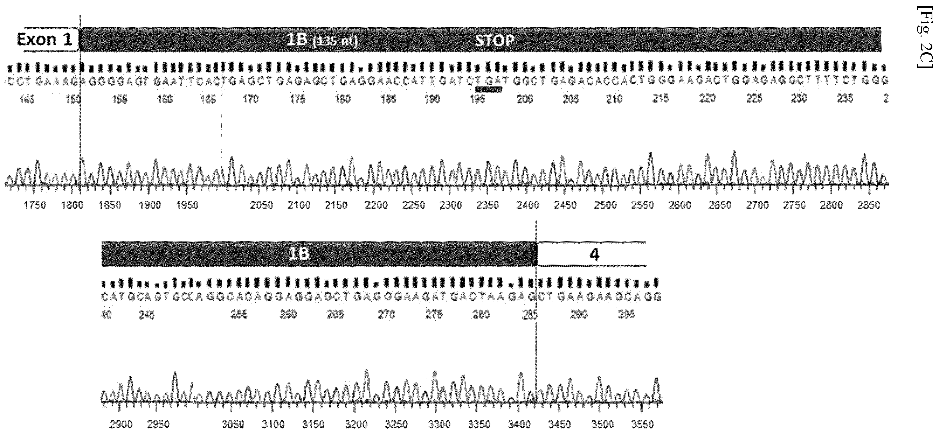

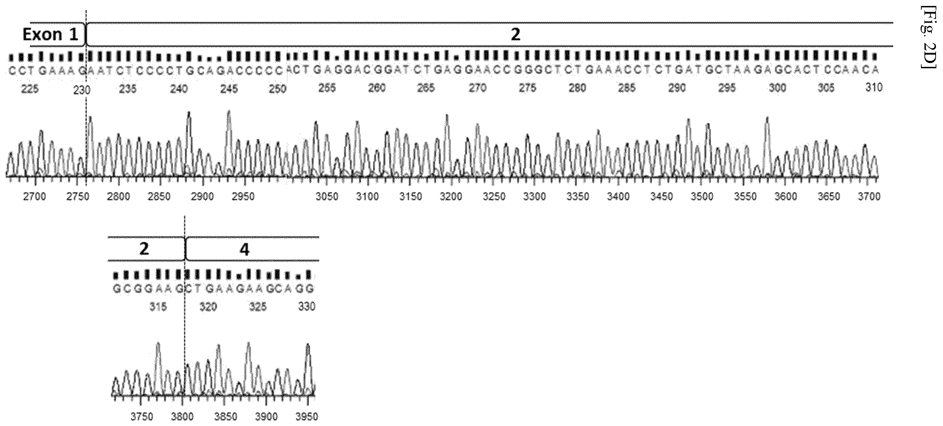

The band at around 335bp observed in RT-PCR of the compound 4-treated sample was extracted, purified, and cloned into a plasmid (Fig. 2A, top, 24 clones). Bands possessing the insert of interest of about 335bp were detected in two clones at around 750bp (Nos. 2 and 10), while bands corresponding to the plasmid not comprising the insert were detected in three clones (450bp, Nos. 6, 7, and 11). Then, the clone No. 2 was analyzed by Sanger sequencing. As shown in the electropherogram in Fig. 2B, exon 1B of 135nt was present following exon 1, and followed by exon 2 of 87nt and joined to it, and then exon 2 was followed by exon 4 and joined to it. Next, the band at around 235bp observed in RT-PCR of the compound 4-treated sample was analyzed in the same way (Fig. 2A, middle, 16 clones). Sanger sequencing of clone 10 of the 16 clones (about 700bp), which was assumed to contain the PCR product of about 235bp to be analyzed, revealed that exon 1B of 135nt was present following exon 1 and followed by exon 4 and joined to it, as shown in Fig. 2C. Finally, the band at around 200bp observed in RT-PCR of the compound 4-treated sample was analyzed in the same way (Fig. 2A, bottom, 24 clones). Sanger sequencing of clone 24, which was assumed to contain the PCR product of about 200bp to be analyzed, revealed that exon 1 was followed by exon 2 and joined to it, and then exon 2 was followed by exon 4 and joined to it, as shown in Fig. 2D. The above results were obtained by single-end analysis from the forward primer side, but the same sequences were confirmed by paired-end analysis from the reverse primer side (data not shown).

### 4. Establishment of qPCR selectively quantifying TAU productive mRNA and TAU nonproductive mRNA

A synthetic DNA corresponding to "TAU nonproductive mRNA," in which 135nt exon 1B was included between exon 1 and exon 4, or "TAU productive mRNA," in which exon 1 and exon 4 were joined, was used as a template, and the designed primers and probes were confirmed to work in qPCR.

### <Methods>

As synthetic DNA templates, 110-TAU and 111-TAU as described in the method section of Experiment 2 and 117-TAU as described below were synthesized (gBLOCKS, IDT). Hereinafter, 110-TAU, 111-TAU, and 117-TAU may be referred to simply as 110, 111, and 117, respectively.
117-TAU pre-mRNA (DNA-standard) (I3-E4): possessing intron 3 followed by a sequence of exon 4.

117 (5'-tctagtaaacaataactgtcttgcttttaccccccttcatttgctgacacatacaccagCTGAAGAAGCAG
GCATTGGAGACACCCCCAGCCTGGAAGACGAAGCTGCTGGTCACGTGACCC
AAG-3') (SEQ ID NO: 27).

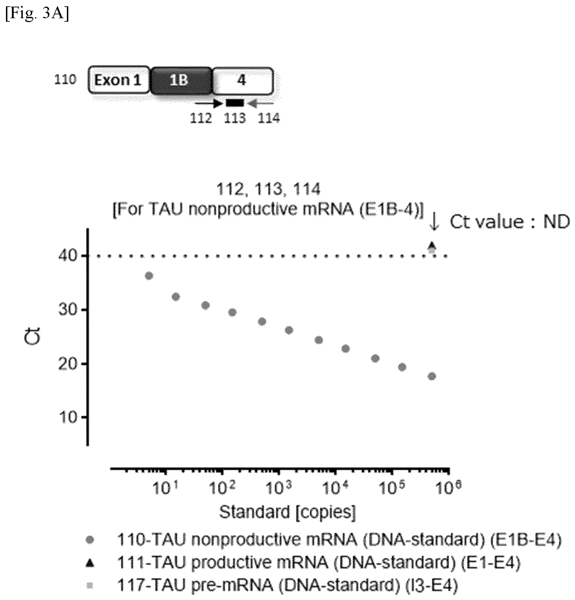

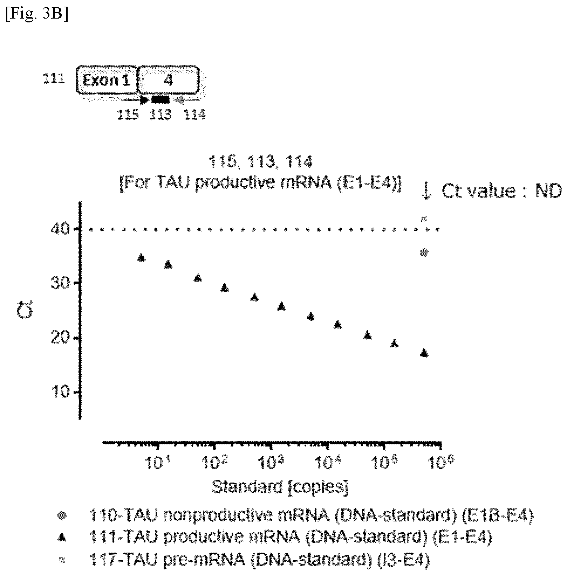

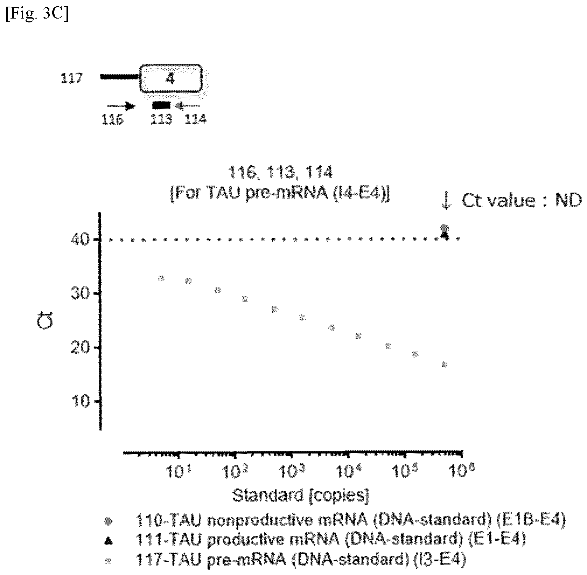

For qPCR, a PCR reaction solution containing the synthetic DNA template (110, 111, or 117), THUNDERBIRD^{®} PROBE qPCR MIX (TOYOBO), qPCR probe (final concentration of 0.15 µM) and primers (final concentration of 0.4 µM) was prepared according to the user's instructions (15 µl of PCR reaction solution was provided). To accurately evaluate the amplification efficiency of PCR and to quantify the copy number as an absolute value, 110, 111, or 117 with known copy number were serially diluted with d.w. containing yeast RNA (final concentration of 10 µg/mL, Invitrogen), and the obtained Ct values were used to generate a calibration curve. The copy number employed to the reaction (x-axis) and the Ct obtained (y-axis) were plotted in the graphs (Figs. 3A, 3B, 3C, bottom). The data was plotted in GraphPad PRISM version 6.07. qPCR was performed in ViiA7 (Applied Biosystems) with initial denaturation: 95°C (1 min), 40 cycles [95°C (15 sec); 60°C (45 sec)]. The following primers and probes were used.
<For TAU nonproductive mRNA detection>
   112 (Fw-Mapt-Exon1B-4) (5'-AGGGAAGATGACTAAGAGCTGA-3') (SEQ ID N O: 28)
   114 (Rv-Mapt-Exon4) (5'-TCACGTGACCAGCAGCTT-3') (SEQ ID NO: 29) 
113 (Probe-Mapt-Exon4) (5'-/56-FAM/AGACACCCC/ZEN/CAGCCTGGAAGA/3IA
BkFQ/-3') (SEQ ID NO: 30)
<For TAU productive mRNA detection>
   115 (Fw-Mapt-Exon1-4) (5'-GCTGGCCTGAAAGCTGA-3') (SEQ ID NO: 31)
   114 (see above)
   113 (see above)
<For TAU pre-mRNA detection>
   116 (Fw-Mapt-Intron3) (5'-CTTCATTTGCTGACACATACACC-3') (SEQ ID NO: 32)
   114 (see above)
   113 (see above)

### <Results>

An absolute quantification system was established for TAU productive mRNA, which was highly expressed and included exon 1 followed by exon 4 and joined it, and TAU nonproductive mRNA, in which exon 1B was included in the TAU productive mRNA. First, as shown in Fig. 3A, qPCR was performed with a set for detecting the TAU nonproductive mRNA including the forward primer 112 designed to span the junction between exon 1B and exon 4. Ct values were not determined even by 40 cycles for the synthetic DNA template 111, which corresponded to productive mRNA including exon 1 followed by exon 4 and joined it without exon 1B, and the synthetic DNA template 117, which corresponded to pre-mRNA including intron 3. In contrast, Ct values were determined for the copy numbers of the synthetic DNA template 110, which corresponded to nonproductive mRNA including exon 1B followed by exon 4 and joined it. Also, the Ct value and the copy number of the DNA template correlated under these conditions, indicating that the copy number of cDNA contained in the reaction solution can be quantified from the Ct value of the targeted sample. The amplification efficiency of PCR was calculated from the slope of the curve to be 94.4%, and cDNA was quantifiable from 5 copies/well, the minimum concentration tested.

Next, to quantify TAU productive mRNA, qPCR was performed with a set for detecting TAU productive mRNA including the forward primer 115 designed to span the junction between exon 1 and exon 4, as shown in Fig. 3B. Ct values could be determined even for low copy numbers with the template 111, which corresponded to productive mRNA. In contrast, with the template 110, which corresponded to nonproductive mRNA, only at the maximum concentration of DNA copies tested (500,000 copies/well), a Ct value of 35.8 was detected, indicating a very slight cross-reactivity. It was considered to be more than 100,000-fold selective for productive mRNA, which was to be quantified. The amplification efficiency of PCR was 91.3% and cDNA was quantifiable from 5 copies/well, the minimum concentration tested.

Furthermore, as shown in Fig. 3C, when qPCR was performed with a set for detecting TAU pre-mRNA utilizing the forward primer 116 designed within intron 3, Ct values were determined only for the synthetic DNA template 117 corresponding to the pre-mRNA. The amplification efficiency of PCR was 98.7% and cDNA was quantifiable from 5copies/well, the minimum concentration tested.

As described above, under the qPCR conditions of Experiment 4, a system capable of selectively detecting and quantifying productive mRNA and nonproductive mRNA from TAU transcripts was successfully established.

### 5. Examination of splice-switching activity of oligonucleotides

To examine the splice-switching activity, productive mRNA and nonproductive mRNA were quantified by qPCR.

### <Methods>

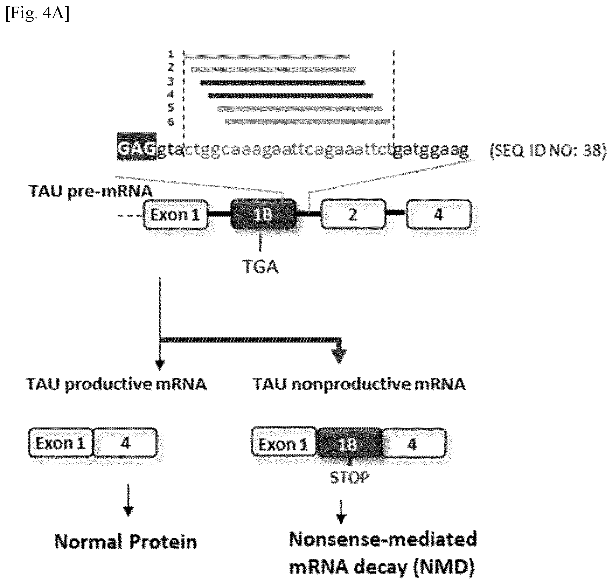

The iPS neurons were treated with each of the six oligonucleotides (compounds 1, 2, 3, 4, 5, and 6) (Fig. 4A) (3 µM, N = 2 wells, data are from two biologically independent samples) through free-uptake according to the culturing method described in the method section of Experiment 2 (15 days treatment) and cDNA samples were prepared from the cells. Non-treated samples were treated with d.w. at a final concentration of 0.1% (N = 6 wells). Then, the amount of TAU cDNA in the iPS neuronal cell sample was quantified according to the qPCR method described in Experiment 4. The quantified value of TAU was normalized for that of ACTB. The ACTB primers and probes were ready-made ones of Hs.PT.39a.22214847 (IDT).
Primer 1 (5'-ACAGAGCCTCGCCTTTG-3') (SEQ ID NO: 20)
Primer 2 (5'-CCTTGCACATGCCGGAG-3') (SEQ ID NO: 21)
Probe (5'-/56-FAM/TCATCCATG/ZEN/GTGAGCTGGCGG/3IABkFQ/-3') (SEQ ID NO: 33)

The synthetic DNA template for the ACTB calibration curve had the sequence of SEQ ID NO: 24 described in the method section of Experiment 2. The results were corrected by adjusting the non-treated group to 100% and plotted on a graph.

### <Results>

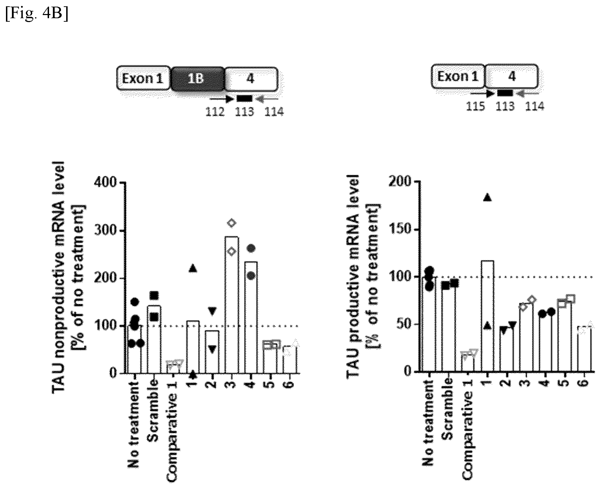

In this experiment, the amounts of change in nonproductive and productive mRNAs with each oligonucleotide were quantified by qPCR. The nonproductive mRNA increased 2.9- and 2.3-fold with compounds 3 and 4, respectively (Fig. 4B, left). In contrast, the productive mRNA was reduced by 28% and 38%, respectively (Fig. 4B, right). From these results, compounds 3 and 4 were found to be effective to modulate TAU splicing. A gapmer-type comparative example compound 1, which was used as a positive control with a different mechanism of action, reduced both nonproductive and productive mRNAs by 82% and 81%, respectively, confirming that comparative example compound 1 reduced TAU by a mechanism other than splice-switching activity.

### 6. Absolute quantification of TAU protein

A system was established to equivalently quantify six different TAU protein variants.

### <Methods>

Homogeneous time resolved fluorescence (HTRF^{®}) assay (64NTAUPEG, Cisbio/Perkin Elmer Japan) was used to quantify the amount of TAU proteins. The followings were performed with reference to the user's instructions. TAU recombinant proteins Tau 441 (2N4R) (SP-495-100) and Tau 352 (0N3R) (SP-497-100) (R&D Systems) were serially diluted. To 1.9 µL of Detection buffer, 0.1 µL of Total-Tau Eu Cryptate Antibody solution was added (Solution A). Next, 0.1 µL of Total-Tau d2 Antibody solution was added to 1.9 µL of Detection buffer (Solution B). Four µL of an antibody solution was prepared by mixing 2 µL each of Solution A and Solution B and added to 16 µL of the solution containing the TAU recombinant protein (MICROPLATE, 384 WELL, PS, F-BOTTOM, SMALL VOLUME, HIBASE, MED. BINDING, WHITE was used for the plate / Greiner Bio-One). The reaction was carried out overnight at 23 to 25°C under light-shielded condition. The excitation was evoked at 320 nm and signals of fluorescence were measured at 620 nm and 665 nm (detection filter) in an HTRF^{®} microplate reader (ARTEMIS). The measured value at 665 nm was divided by the measured value at 620 nm and multiplied by 10,000 and plotted on the vertical axis (N = 2 wells) on the graph. On the horizontal axis, the molar concentration of the recombinant protein calculated from the concentration of the protein and the molecular weight of total amino acids was plotted. The graph was prepared by GraphPad PRISM version 6.07.

### <Results>

Since there were six splicing variants of TAU productive mRNA in total in the central nervous system (Wang JZ et al., Prog Neurobiol. 85. 148-75. 2008), six TAU proteins having different molecular weights were expected to be expressed (Fig. 5, top). In this experiment, it was evaluated whether the HTRF^{®} kit commercially available from Cisbio/Perkin-Elmer Japan, which did not disclose details of the anti-TAU antibody, recognized all TAU proteins equally. The TAU recombination protein derived from the longest TAU productive mRNA (2N4R TAU/441 a.a.) and the TAU recombination protein derived from the shortest TAU productive mRNA (0N3R TAU/352 a.a.) were measured at various concentrations, and both were detected equally (Fig. 5, bottom).

### 7. Study of concentration dependency (I)

The oligonucleotide-concentration dependency was studied. Productive and nonproductive mRNAs and TAU protein were quantified to examine the splice-switching activity and the TAU protein knockdown activity in details.

### <Methods>

The iPS neurons were treated with an SSO, compound 7 (having the same sequence as compound 3) or compound 8 (having the same sequence as compound 4), or a gapmer-type oligonucleotide, comparative example compound 1, through free-uptake according to the culturing method described in the method section of Experiment 2 (15 days treatment) and then the cells were lysed with RIPA Buffer (1x conc. ready-to-use solution, protease inhibitor-free). To the resulting product, a reverse transcriptase was added to synthesize a cDNA sample. A non-treated sample was prepared from cells treated with d.w. at a final concentration of 0.1 %. Then, the amounts of TAU and ACTB cDNAs were quantified according to the qPCR method described in Experiment 4. Only the values of wells in which the quantified value of ACTB fell within the range of "the mean of all measurements ± 2-fold standard deviation" were analyzed. Neurofilament light polypeptide (NfL) was quantified as a control. For NfL, the following primers, probe and synthetic DNA template for a calibration curve were used (IDT). The quantified values of TAU and NfL were corrected for that of ACTB. The results were plotted on a graph by adjusting the non-treated group to 100%.

### <For NfL mRNA detection>

Primer (5'-CCATCAGCAACGACCTCAA-3') (SEQ ID NO: 34)
Primer (5'-GCTTCCAGGACCTTGTTCT-3') (SEQ ID NO: 35)
Probe (5'-/56-FAM/CTTCGCCAG/ZEN/CTTCATCGAGCG/3IABkFQ/-3') (SEQ ID NO: 36)

NfL (5'-CGACCTGAGCCAGGTAGCCGCCATCAGCAACGACCTCAAGTCCATC
CGCACGCAGGAGAAGGCGCAGCTCCAGGACCTCAATGACCGCTTCGCCAGC
TTCATCGAGCGCGTGCACGAGCTGGAGCAGCAGAACAAGGTCCTGGAAGCC
GAGCTGCTGGTGCTGCGCCAGAAGCACTCC-3') (SEQ ID NO: 37)

Next, to measure the total protein content with the BCA kit (Nacalai Tesque), 2 µL of the lysate solubilized with RIPA Buffer (1x ready-to-use solution; protease inhibitor-free) was mixed with 8 µL of d.w., and the resulting product was mixed with 200 µL of BCA solution (mixture of 4 µL of Solution B and 196 µL of Solution A) and incubated at 60°C for about 30 minutes. A calibration curve for total protein was prepared with BSA solutions serially diluted from 500 ng/mL by using the Quick Start Bovine Serum Albumin Standard Set (BIO-RAD). The absorbance was measured at 562 nm to quantify the total protein content of the lysate. Then, to measure the concentration of TAU protein with HTRF^{®} assay, the lysate solubilized with RIPA Buffer (1x ready-to-use solution, protease inhibitor-free) was diluted 40-fold with 1X RIPA Buffer with Protease Inhibitor Cocktail (Nacalai Tesque) so that the resulting solution was added to a well at 15 µL/well, and the antibody solution (an equal volume mixture of Solution A and Solution B described in the method section of Experiment 6) was added thereto at 4 µL/well. To prepare a calibration curve for TAU protein, Tau 441 (2N4R) (SP-495-100, R&D Systems) serially diluted with RIPA buffer was used. The reaction was carried out overnight at 23 to 25°C under light-shielded condition. The excitation was evoked at 320 nm and the signals of fluorescence were measured at 620 nm and 665 nm (detection filter) in an HTRF^{®} microplate reader (ARTEMIS). The measured value at 665 nm was divided by the measured value at 620 nm and multiplied by 10,000, and from the obtained value and the calibration curve for Tau 441 (2N4R), the concentration of TAU protein in the iPS neuron-derived lysate was determined. A value obtained by dividing the value of TAU protein quantification by the value of total protein quantification by BCA was plotted on a graph after being corrected by adjusting the non-treated group to 100%. The graph was provided by GraphPad PRISM version 6.07.

### <Results>

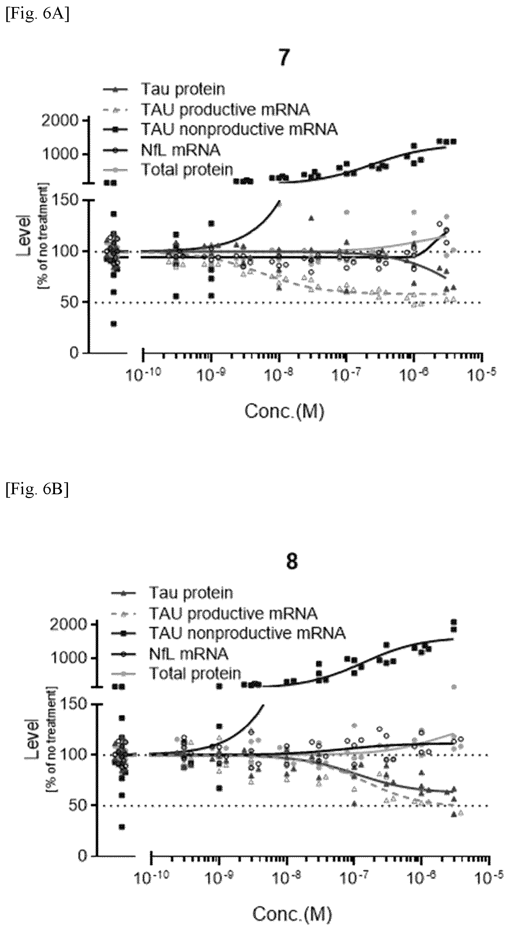

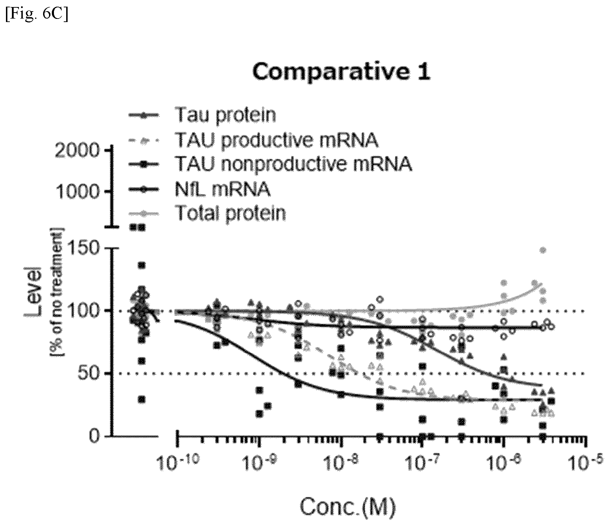

When the concentration dependency was evaluated with compound 7 (having the same sequence as compound 3) or compound 8 (having the same sequence as compound 4), which comprises different modified oligonucleotides, TAU nonproductive mRNA increased at a concentration in the order of nM or more and TAU productive mRNA decreased at about 10 nM or more (Figs. 6A and 6B). In addition, compounds 7 and 8 reduced the amount of TAU protein (Figs. 6A and 6B). In contrast, both nonproductive mRNA and productive mRNA decreased at a concentration in the order of nM or more with the gapmer-type comparative example compound 1 (Fig. 6C). The lack of NfL reduction with any of the oligonucleotides indicated that compounds 7 and 8 had the potential to selectively reduce TAU productive mRNA.

### 8. Identification of neuronal cells with increased TAU nonproductive mRNA

Cells affected by the SSO were confirmed by cell staining.

### <Methods>

The iPS neurons were treated with the SSO compound 7 (N = 2 wells) or compound 8 (N = 2 wells) through free-uptake according to the culturing method described in the method section of Experiment 2 (15 days treatment) and the cells were fixed in 10% neutral buffered formalin solution for about 16 hours at room temperature. In this experiment, to reduce cell detachment due to the fixation, the coating agent in the method of Experiment 2, poly-L-omithine (Sigma-Aldrich), was changed to 0.1% polyethyleneimine solution. Also, to increase the percentage of inhibitory neurons, iCell^{®} GABAergic neurons (Fujifilm Cellular Dynamics, Inc.) and iCell GlutaNeurons 01279 were mixed at a ratio about 20:80 and then seeded. The TAU nonproductive mRNA was visualized according to the user's instructions of Target Probe-BA-Hs-MAPT-211-2EJ-O1-C1 (1201211-C1) and BaseScope^{™} Reagent Kit v2- RED (323900) purchased from Advanced Cell Diagnostics, Inc. (ADC). Specifically, fixed cells were washed with D-PBS (-) (PBS, FUJIFILM Wako Pure Chemical Corporation) and then digested with Protease plus diluted 15-fold with PBS for 10 minutes at room temperature. Then, after washed with PBS, the sample was reacted with the probe at 40°C for 2 hours. After washed twice with 1x wash buffer, the sample was reacted with the AMP1 reagent at 40°C for 15 to 30 minutes. This procedure was repeated for AMPs 2, 3, 4, 5, and 6. Then, after washed twice with 1x wash buffer, the sample was reacted with AMP7 for 15 to 30 minutes at room temperature. This procedure was then also performed with AMP8. After washed twice with 1x wash buffer, the sample was reacted with the chromogenic reagent BaseScope^{™} Fast RED solution containing BaseScope^{™} Fast RED-B (obtained by diluting Solution B 60-fold with Solution A) for about 30 minutes at room temperature. After washed with PBS, the sample was stored at 4°C under light-shielded condition.

Next, the following procedures were performed to immune-stain the cells with antibodies. After washed with PBS, the cells were permeabilized with 0.1% TritonX-100-containing PBS solution for 10 minutes at room temperature. After washed with PBS, the cells were treated with Pierce^{™} Protein-Free T20 (TBS) Blocking Buffer (Thermo Fisher Scientific) for 1 hour at room temperature for blocking. After washed with PBS, the sample was reacted for 1 hour at room temperature with an anti-Neuronal nuclei antigen (NeuN) antibody (ab104224, Abcam) and an anti-Gamma-Aminobutyric Acid (GABA) antibody (A2052-100UL, Sigma-Aldrich), each diluted 1,000-fold with the blocking reagent. After washed with PBS, the sample was reacted for 1 hour at room temperature with secondary antibodies, Donkey Anti-Mouse IgG H&L (Alexa Fluor^{®} 488) (ab150105, Abcam) and Donkey Anti-Rabbit IgG H&L (Alexa Fluor^{®} 647) (ab150075, Abcam), each diluted 1,000-fold with the blocking reagent, and DAPI (DOJINDO LABORATORIES), diluted 10,000-fold with the blocking reagent. The cells were washed with PBS and excited at 405 nm, 488 nm, 561 nm, and 640 nm on a high-throughput cell function discovery system CellVoyager CV8000 (YOKOGAWA), and the signals were detected with bandpass filters of 445/45 nm, 525/50 nm, 600/37 nm, and 676/29 nm (signals derived from DAPI, NeuN, TAU nonproductive mRNA, and GABA, respectively). Images were output with a high-content analysis software CellPathfinder.

### <Results>

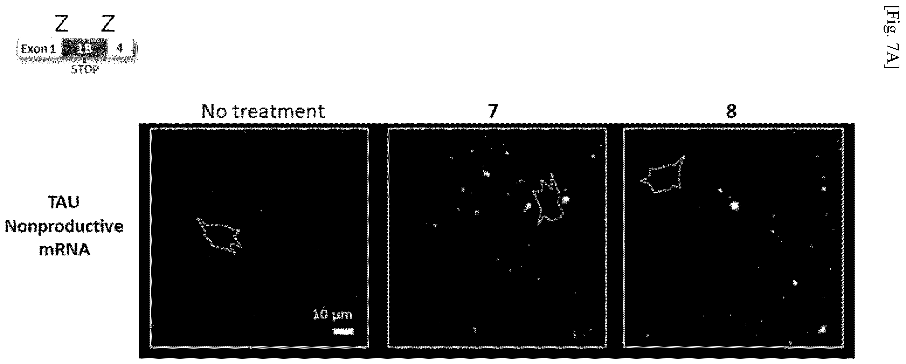

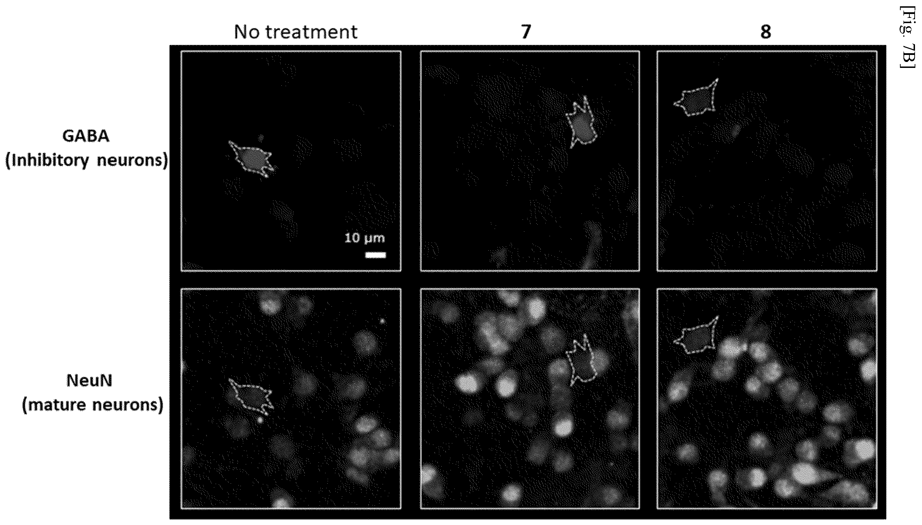

To prepare probes to detect TAU nonproductive mRNA, probes which hybridize the junction of exon 1 and exon 1B and the junction of exon 1B and exon 4 were synthesized at ACD (Fig. 7A, top). TAU nonproductive mRNA (Fig. 7A, bottom) and NeuN protein and GABA (Fig. 7B) were visualized by fluorescent staining. Compound 7 or 8 increased TAU nonproductive mRNA-positive cells compared with no treatment (Fig. 7A, bottom). The cells with TAU nonproductive mRNA were found predominantly in NeuN-positive and GABA-negative cells (Fig. 7B), indicating that TAU nonproductive mRNA tended to increase in excitatory neurons.

### 9. Single cell RNA-seq

Single cell RNA sequencing (scRNA-seq) was performed to confirm SSO-induced changes in TAU (MAPT) expression in excitatory and inhibitory neurons separately.

### <Methods>

The iPS neurons were treated with the SSO compound 7 or compound 8 or the comparative example compound 1 through free-uptake according to the culturing method described in the method section of Experiment 2 (17 days treatment) and the neuronal cells were dispersed and collected by using Papain Dissociation System (Worthington Biochemical Corporation). To increase the percentage of inhibitory neurons, iCell^{®} GABAergic neurons (FUJIFILM Cellular Dynamics, Inc.) and iCell GlutaNeurons 01279 were mixed and seeded. The plate used was Corning^{®} BioCoat^{®} poly-D lysine 96-well Clear Flat Bottom TC-treated Microplate (Corinig), and 130,000 cells/well of GlutaNeurons and 42,900 cells/well of GABAergic neurons were seeded. The cells were cultured with a medium of 200 µL/well and the half of the medium was changed every 3 days. On day 18 of culture, a digestive solution of papain (vial 2) mixed with Accumax and DNase was added to the neurons and the cells were dispersed at 37°C for 30 minutes under 5% CO₂. The wells were washed with a resuspension solution (DNase, vial 4, and HBSS containing 5 µM Y-27632) and the collected cell suspension was filtrated with a pluriStrainer-Mini 40 µm (pluriSelect USA) at 500 x g for 5 minutes to remove debris. After the supernatant was removed, the precipitated cells were suspended with the resuspension solution and 5 times the volume of vial 4 was added to the lower layer, and then the cells were centrifuged at 500 x g for 5 min. The supernatant was removed and the precipitated cells were suspended in PBS containing 0.04% BSA (Miltenyi Biotec). After the cells were centrifuged at 500 x g for 5 minutes and the supernatant was removed, the precipitated cells were resuspended with 0.04% BSA-containing PBS to count live cells. A suspension was prepared according to Chromium Single Cell 3' Reagent Kits User Guide (v3. 1 Chemistry Dual Index, 10x Genomics) by using Next GEM Single Cell 3' GEM Kit v3.1, Next GEM Single Cell 3' Library Kit v3.1, and Single Index Kit T Set A (10x Genomics), and with Chromium controller (10x Genomics) at 10,000 live cells/reaction. The reverse transcription reaction was performed at 37°C (15 min), 53°C (45 min), and 85°C (5 min) at 50 µL/well to obtain cDNA solutions (C1000 Touch thermal cycler, Bio-Rad Laboratories, Inc.). The cycles of PCR reactions to amplify cDNA and GEX libraries were 11 and 14 cycles, respectively. The quality of both libraries and fragment size was measured on High Sensitivity D5000 ScreenTape (Agilent) and TapeStation (Agilent). Read lengths of 28bp for R1 and 100bp for R2 were sequenced by DNBSEQ-G400 sequencing system (MGI Tech). More than 400 million reads per sample were quality-assessed using FastQC (v. 0.11.9). The sequence data was processed with Cellranger 7.0.1 and read mapping was performed with default parameters and the GRCh38 reference genome. The analysis was performed with the Seurat package (version 3.1.4) in R (version 3.6.1) for the count matrix calculated by Cellranger. The percentage of mitochondrial genes among the RNA molecules detected in each cell was calculated. By extracting cells with a detected number of RNA molecules greater than 2500 and a mitochondrial gene percentage of less than 10%, cells with particularly low quality of gene expression measurement were excluded. Expression normalization was then performed and data from four samples (non-treated group, compound 7-treated group, compound 8-treated group, and comparative example compound 1-treated group) were combined. Within the combined data, 2,000 genes with high variance in expression level were selected and standardized for expression level. Principal Component Analysis (PCA) was performed using 2,000 standardized genes, and the top 30 components were used for dimensionality compression by Uniform Manifold Approximation and Projection (UMAP) and clustering. From the number of gene detections per cluster, the population with low quality of gene expression measurement was identified and excluded. The population of excitatory neurons was presumed from the expression pattern of SLC17A6. The population of inhibitory neurons was estimated from the expression patterns of GAD1 and GAD2. Genes specific to each cluster were also extracted. The cells were classified into excitatory, inhibitory, ISL1 high-expressing, and LHX8 high-expressing cells, and the average gene expression levels for each classification were determined and a heat map was provided for the expression levels of SLC17A6, TBR1, GAD1, GAD2, ISL1, PCDH11Y, LHX8, and CRABP1. The estimated population of excitatory neurons was selected and the expression levels of TAU (MAPT) between the non-treated and oligonucleotide-treated groups were compared. The presumed population of inhibitory neurons was selected and the expression levels of TAU (MAPT) between the non-treated and oligonucleotide-treated groups were compared. The delta in the percentage of reduced TAU (MAPT) expression between excitatory and inhibitory neurons in each oligonucleotide treated group was calculated. One hundred cells that were allowed to overlap were randomly selected from each of the non-treated and oligonucleotide-treated groups, and the difference in TAU expression between each cell was determined. This trial was repeated 10 times according to the bootstrap method, and the mean and 95% confidence interval were calculated by using SAS Software for Windows, Release 9.4 (SAS Institute Japan Ltd.).

### <Results>

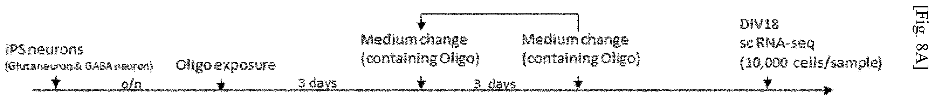

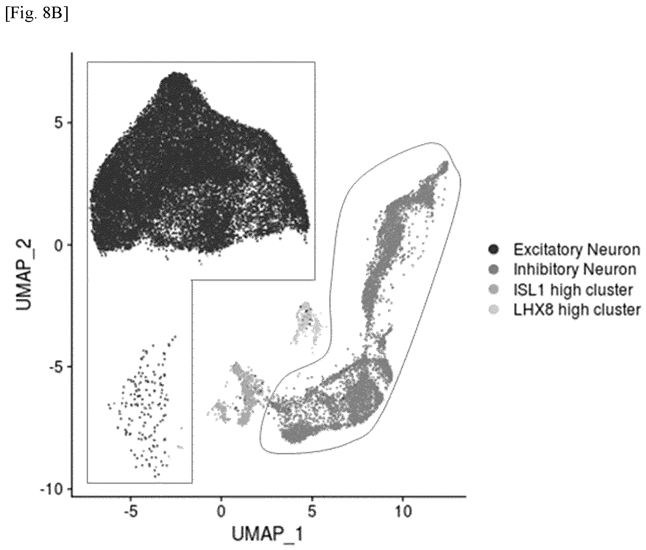

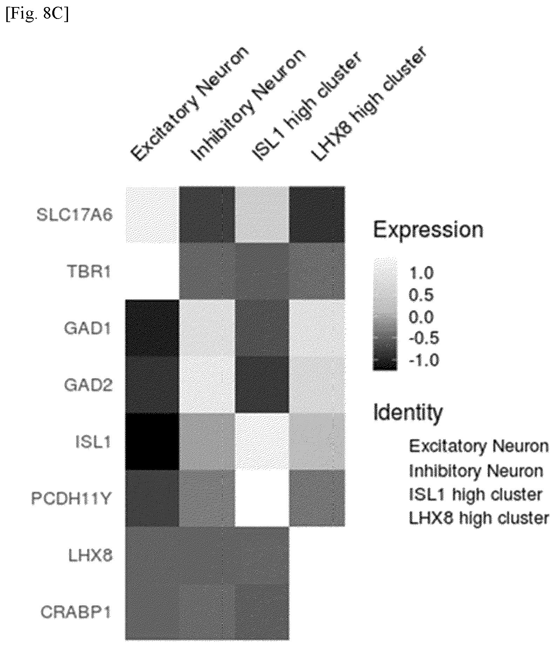

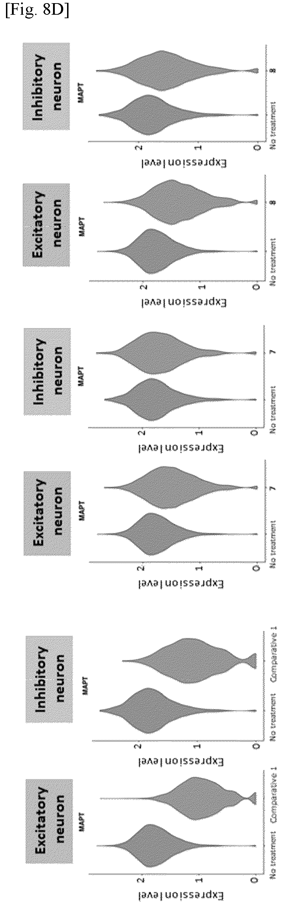

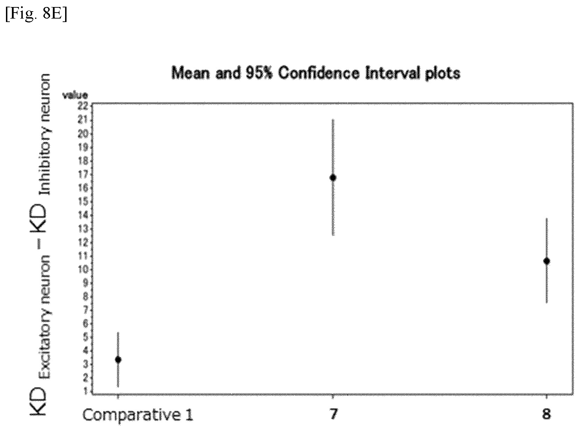

Changes in TAU expression in the populations of excitatory and inhibitory neurons were assessed by scRNA-seq (Fig. 8A). The results of clustering into two dimensions by compressing dimensions in the gene expression profiles of each cell are shown in Fig. 8B. The left population (boxed population) was defined as a population of excitatory neurons because the expression of SLC17A6/VGLUT2, a transporter for uptaking glutamate into cells, was observed (Figs. 8B, C). The lower right population (circled population) was defined as a population of inhibitory neurons because it expressed GAD1 and GAD2, enzymes involved in GABA production (Figs. 8B, C). The mean change in TAU (MAPT) expression (knockdown (KD) level) in the population of excitatory neurons of the comparative example compound 1-treated group was calculated to be 54.2% lower than that of the non-treated group (Fig. 8D, leftmost panel). Then, the KD level in the population of inhibitory neurons of the comparative example compound 1-treated group was calculated to be 49.4% lower than that of the non-treated group (Fig. 8D, second from the left). These results showed that the delta between the KD levels of the two populations treated with comparative example compound 1 was 4.8% (4.8% = 54.2% - 49.4%). Compound 7 reduced TAU by 20.4% in the population of excitatory neurons and by 6.4% in the population of inhibitory neurons. Thus the delta between the KD levels of the two populations treated with compound 7 was 14.0% (14. 0% = 20.4% - 6.4%) (Fig. 8D, third and fourth from left), which was greater than that treated with comparative example compound 1. Compound 8 reduced TAU by 29.0% in the population of excitatory neurons and by 18.2% in the population of inhibitory neurons. Thus the delta between the KD levels of the two populations treated with compound 8 was 10.8% (10. 8% = 29.0% - 18.2%) (Fig. 8D, fifth and sixth from left), which was greater than that treated with comparative example compound 1. From there results, the delta between the KD levels of the both populations was greater with compounds 7 and 8 than that with comparative example compound 1, indicating a potent reduction in TAU in excitatory neurons than in inhibitory neurons. Then, 100 cells were randomly selected from the non-treated and oligonucleotide-treated groups, respectively, and the delta in KD level was determined. This trial was repeated 10 times according to the bootstrap method, and the mean and 95% confidence interval were calculated, confirming a statistically significant difference (5% significance level) between comparative example compound 1 and compound 7 or 8 (Fig. 8E). These results demonstrated that compounds 7 and 8 significantly and selectively reduced TAU in the population of excitatory neurons compared to comparative example compound 1.

### 10. Attenuation of hyper synchronization of neural activity by reduction of endogenous TAU protein

It was evaluated whether SSO-induced reduction of endogenous TAU protein attenuated hyper synchronization of neural activity.

### <Methods>

The Maestro Pro Multi Electrode Array (MEA) was performed according to the user's instructions of iCell GlutaNeurons 01279 (FUJIFILM Cellular Dynamics, Inc.). Specifically, a plate Cyto View MEA 24 (Axion BioSystems) was coated with 0.1% polyethyleneimine solution overnight at room temperature, washed with d.w., and dried. Then, the plate was further coated overnight at 4°C with a medium containing 100 µg/mL laminin (Sigma-Aldrich). On the next day, the plate was placed in a 5% CO₂ incubator (37°C) for another 1 hour. Equal numbers of iCell^{®} GABAergic neurons and iCell GlutaNeurons 01279 were mixed and seeded to the recording electrode area of the well at 9.9 x 10⁵ cells/cm². On the next day, XCL-1 Mature Astrocytes (AM-001-1V) were added to the recording electrode area of the well at 2.2 x 10⁵ cells/cm². Two days later, comparative example compound 1 or compound 7 was diluted with the medium to provide a given concentration and added to the cells (600 µL/well). The non-treated group was cultured in the medium. Each group had 6 wells (N = 6). The half of the medium was changed every 4 days. On day 25 of culture, before addition of KCl, spontaneous nerve activity in pretreatment (pre) was measured with Maestro Pro (Axion BioSystems) at 37°C, 5% CO₂ for 15 minutes. To induce hyper synchronization of neural activity, 3 µL of 1 M KCl was added to 600 µL/well of the culture medium so that about 10 mM KCl was exposed (shown as <10 mM, because it was only disclosed that the BrainPhys medium contained less than 5 mM KCl at the basal condition and the final concentration of KCl could not be accurately determined.) (Bardy C et al., Proc Natl Acad Sci U S A. 2015. 112. E2725-E2734.). AxIS Navigator (version2.0.4.21) and Neural Metric Tool (version2.4.12) were used for the analysis. To detect the network bursts, in the Burst Detector Setting, the Burst Detection Algorithm was set to Inter-Spike Interval (ISI) Threshold, Minimum Number of Spikes (network bursts) was set to 50, Maximum Inter-Spike Interval (network bursts) was set to 100 ms, and Minimum Participating Electrodes (%) was set to 35 (default setting).

### <Results>

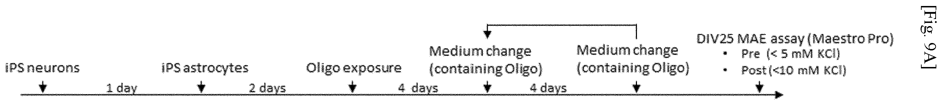

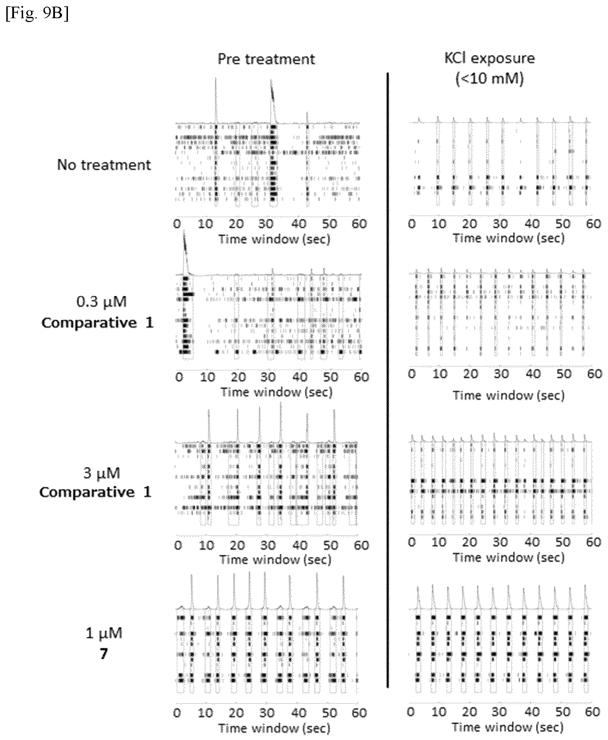

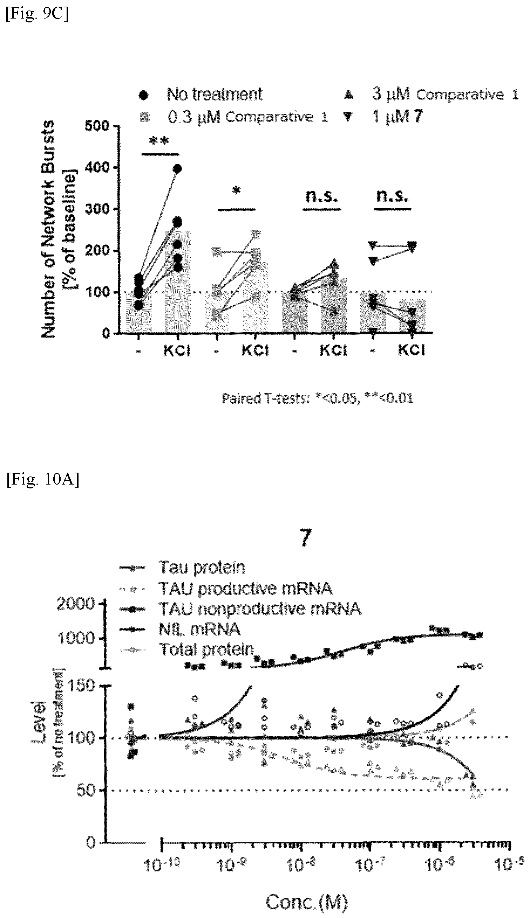

After knockdown of endogenous TAU with two oligonucleotides over about 22 days as shown in Fig. 9A, spontaneous neuronal activity in Pre was determined. Spontaneous neural activity was observed with or without the oligonucleotide treatment (Fig. 9B, left). KCl was then added to induce pathological hyper synchronization of neural activity. The group without the oligonucleotide treatment (no treatment) showed a significant increase in the number of network bursts, which are represented in boxes (Fig. 9B, right, and Fig. 9C). The term network bursts refer to a phenomenon in which neural activities are recorded simultaneously at multiple electrodes in a single well (definition of detection was given in the method section). We considered that a prolonged induction of network bursts may cause neurodegeneration, and examined whether knockdown of the endogenous TAU could attenuate the abnormal increase in network bursts. The increase was not suppressed by pretreatment with 0.3 µM of comparative example compound 1, but mitigated by pretreatment with 3 µM of comparative example compound 1 (Fig. 9C). Also, the increase in network bursts was attenuated with 1 µM of compound 7 (Fig. 9C). These results demonstrated that the reduction in TAU productive mRNA through the increase in TAU nonproductive mRNA by compound 7 could also attenuate hyper synchronization of neural activity.

### 11. Study of concentration dependency (II)

The oligonucleotide-concentration dependency was studied for SSO-type and gapmer-type oligonucleotides possessing the same sequence. Productive and nonproductive mRNAs and TAU protein were quantified to examine the splice-swithing activity and the TAU protein knockdown activity in details.

### <Methods>

The iPS neurons were treated with an SSO, compound 7 or compound 8, or a gapmer-type oligonucleotide corresponding to each SSO, comparative example compound 2 or 3, through free-uptake according to the culturing method described in the method section of Experiment 2 (15 days treatment). Non-treated samples were treated with d.w. at a final concentration of 0.1%. The comparative example compounds 2 and 3 were synthesized in the same way as in Experiment 1.

**Table 3**

| Compound No. | Sequence (5'→3') | SEQ ID NO: |
|---|---|---|
| **7** | A_{As}TₙT_{As}T_{dn}^{m}C_{As}T_{ds}G_{As}A_{ds}A_{As}T_{ds}T_{As}^{m}C_{ds}T_{As}T_{dn}T_{As}G_{dn}^{m}C_{As}^{m}C_{d} | 16 |
| **8** | A_{As}A_{dn}T_{As}T_{dn}T_{As}^{m}C_{ds}T_{As}G_{ds}A_{As}A_{ds}T_{As}T_{ds}^{m}C_{As}T_{dn}T_{As}T_{dn}G_{As}^{m}C_{d} | 17 |
| Comparative example compound 2 | A_{Ls}T_{Ls}T_{Ls}T_{Ls}C_{ds}T_{ds}G_{ds}A_{ds}A_{ds}T_{ds}T_{ds}C_{ds}T_{ds}T_{ds}T_{Ls}G_{Ls}C_{Ls}C_{L} | 39 |
| Comparative example compound 3 | A_{Ls}A_{Ls}T_{Ls}T_{Ls}T_{ds}C_{ds}T_{ds}G_{ds}A_{ds}A_{ds}T_{ds}T_{ds}C_{ds}T_{ds}T_{Ls}T_{Ls}G_{Ls}C_{L} | 40 |

| | | |
|---|---|---|
| "d" = DNA, "L" = LNA, "A" = AmNA, "s" = phosphorothioate, "n" = phosphoramidate, "^{m}C" = 5-methylcytosine. | | |

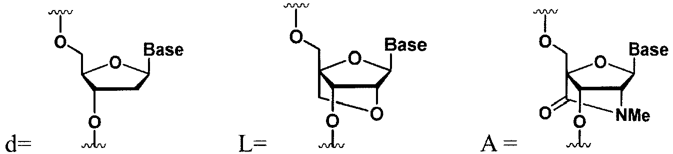

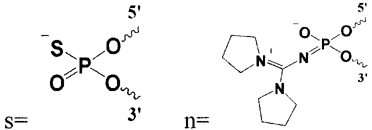

To perform qPCR, according to the user's instructions of SuperPrep^{®} II Cell Lysis & RT Kit for qPCR (TOYOBO), 10 µL of a lysis buffer (a mixture of 9.78 µL of Lysis Solution, 0.17 µL of RNase Inhibitor, and 0.05 µL of gDNA Remover) was added to the cells and the mixture was incubated at room temperature for 5 minutes to degrade the genomic DNA and obtain an RNA-containing solution. Then, for reverse transcription, 5X RT Master Mix, the RNA-containing solution and d.w. were mixed and reacted at 37°C (15 min), 50°C (5 min) and 98°C (5 min) to obtain a cDNA solution. The cDNA solution was then diluted 4-fold with d.w. Then, the amounts of TAU, ACTB, and NfL were quantified according to the qPCR method described in Experiment 4. The results were plotted on a graph by adjusting the non-treated group to 100%.

To quantify the amount of TAU protein, the cells were lysed with RIPA Buffer (1x conc. ready-to-use solution; protease inhibitor-free). According to the method described in Experiment 7, the total protein content and the concentration of TAU protein were measured with the BCA kit (Nacalai Tesque) and HTRF^{®} kit. In EnVision multimode plate reader (Perkinelmer), the excitation was evoked at 320 nm and signals of fluorescence were measured at 620 nm and 665 nm (detection filter). A value obtained by dividing the value of TAU protein quantification by the value of total protein quantification by BCA was plotted on a graph after being corrected by adjusting the non-treated group to 100%.

### <Results>

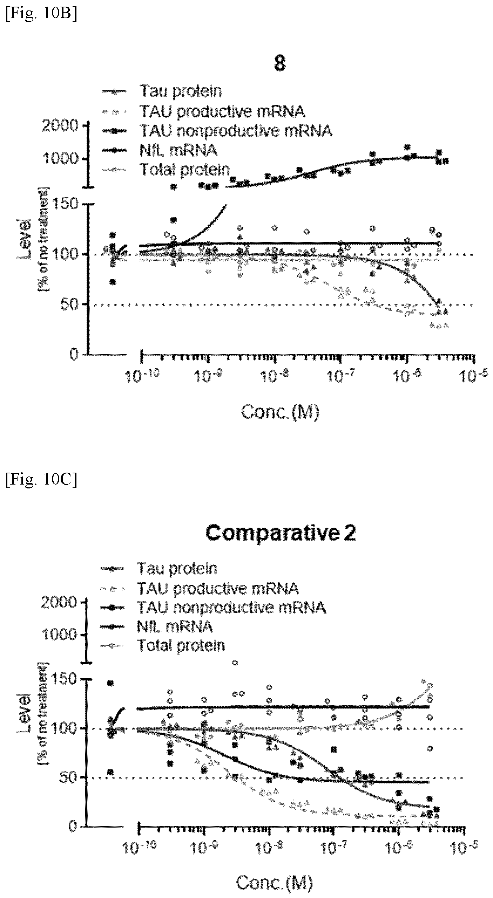

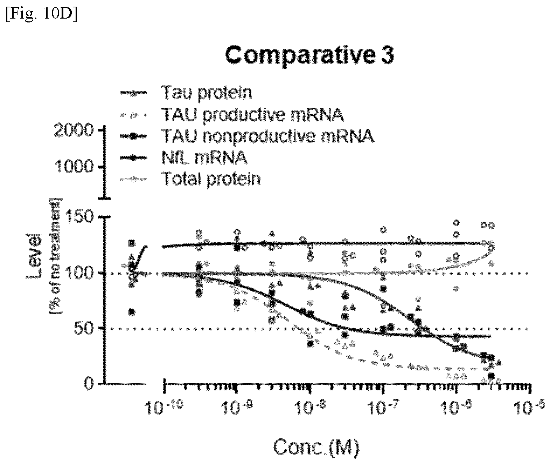

With the SSO compound 7 or compound 8, TAU nonproductive mRNA increased at a concentration in the order of nM or more and TAU productive mRNA decreased at about 10 nM or more (Figs. 10A and 10B). In contrast, both nonproductive mRNA and productive mRNA decreased at a concentration in the order of nM or more with the gapmer-type comparative example compound 2 or 3, which had the identical sequence as compound 7 or compound 8 (Fig. 10C and 10D). These results confirmed that the SSO-type oligonucleotide of the present disclosure selectively decreases productive mRNA through splicing, whereas a gapmer-type oligonucleotide decreases mRNA regardless of whether it is nonproductive or productive mRNA, even though the gapmer-type oligonucleotide has the identical sequence as the SSO-type oligonucleotide.

The above experiments demonstrated that SSOs that increase TAU nonproductive mRNA including exon 1B reduce the amount of TAU protein, and that this effect is suggested to be selective to excitatory neurons and can attenuates hyper synchronization of neural activity. The SSOs are useful in the treatment or prevention of TAU-related diseases.

## Claims

1. A splice-switching oligonucleotide that binds to intron 1B of TAU pre-mRNA.

2. The splice-switching oligonucleotide according to claim 1, which is capable of increasing an amount of TAU mRNA including TAU exon 1B.

3. The splice-switching oligonucleotide according to claim 1, which is capable of reducing an amount of TAU protein.

4. The splice-switching oligonucleotide according to claim 1, which comprises a sequence complementary to a part of intron 1B of TAU pre-mRNA.

5. The splice-switching oligonucleotide according to claim 1, which comprises a sequence complementary to at least a part of the sequence at positions 6 to 24 of SEQ ID NO: 2.

6. The splice-switching oligonucleotide according to claim 1, which is 16 to 20 bases in length.

7. The splice-switching oligonucleotide according to claim 1, which is capable of reducing an amount of TAU protein selectively in an excitatory neuron.

8. The splice-switching oligonucleotide according to claim 7, which is capable of reducing an amount of TAU protein in an excitatory neuron by at least 5% greater than in an inhibitory neuron.

9. A pharmaceutical composition comprising the splice-switching oligonucleotide according to any one of claims 1 to 8 for treating or preventing a TAU-related disease.

10. The pharmaceutical composition according to claim 9, wherein the TAU-related disease is tauopathy or epilepsy.

11. The pharmaceutical composition according to claim 9, wherein the TAU-related disease is selected from the group consisting of Alzheimer's dementia, vascular dementia, dementia with Lewy bodies, Parkinson's disease, amyotrophic lateral sclerosis, Down syndrome, Pick's disease, chronic traumatic encephalopathy, progressive supranuclear palsy, corticobasal degeneration, and myotonic dystrophy type 1.

12. The pharmaceutical composition according to claim 9, wherein the TAU-related disease is selected from the group consisting of Dravet syndrome, tuberous sclerosis complex, episodic ataxia, CDFE syndrome, and DEE14 or 18.

13. The pharmaceutical composition according to claim 9, wherein the TAU-related disease is selected from the group consisting of glaucoma and age-related macular degeneration.
